# EUROPEAN PATENT SPECIFICATION

(11) **EP 1 389 984 B1**
(45) Date of publication and mention of the grant of the patent: **11.03.2009**
(21) Application number: 02739531.8
(22) Date of filing: 29.05.2002
(51) Int. Cl.: A61F 5/00, A61B 17/064, A61B 19/00

(54) **OBESITY TREATMENT TOOLS**
VORRICHTUNGEN ZUR BEHANDLUNG VON FETTLEIBIGKEIT
INSTRUMENTS DE TRAITEMENT DE L'OBESITE

(30) Priority: 30.05.2001 US 871297; 23.05.2002 US 155362
(43) Date of publication of application: 25.02.2004
(73) Proprietor: Satiety, Inc., Palo Alto, CA 94303-3313 (US)
(72) Inventor: WELLER, Gary, Los Gatos, CA 95032 (US); GERBI, Craig, Mountain View, CA 94041 (US); GANNOE, James, Menlo Park, CA 94025 (US); DEEM, Mark, E., Mountain View, CA 94041 (US); SUTTON, Douglas, S., Pacifica, CA 94044 (US); GIFFORD, Hanson, S., III, Woodside, CA 94062 (US); ANDREAS, Bernard, H., Redwood City, CA 94062 (US); FRENCH, Ronald, G., Santa Clara, CA 95050 (US)
(74) Representative: Price, Nigel John King
(86) International application number: PCT/US2002/017077
(87) International publication number: WO 2002/096327

(56) References cited:
- WO-A-00/07640
- WO-A-00/78227
- WO-A-01/26557
- WO-A-01/28432
- FR-A- 2 768 324
- US-A- 6 165 183

## Description

The present invention relates generally to tools for the treatment of obesity. More particularly, the present invention relates to tools for performing less traumatic gastroplasty procedures.

Obesity is considered a major health problem with annual associated costs reaching $100 billion in the U.S. alone. Morbid obesity is a condition of obesity with the presence of a secondary debilitating progressive disease and is generally associated with a body mass index (BMI) ≥40 kg/m². While the basic mechanism of obesity is simply an imbalance between caloric intake and burn rate, the underlying factors are varied and complex and conservative attempts at sustained weight loss with this population are almost always unsuccessful. Often, there are genetic and other biological influences that may override environmental causes. Consequently, obesity.is a disease that eludes a simple treatment, with a recurrence rate above 90% for those who attempt to lose weight. Moreover, long-term results using conservative treatments for morbid obesity are generally unsuccessful and are typically associated with further loss of self-esteem with the regaining of weight. Hypertension, cardiovascular disease, diabetes, along with a host of other cornorbidities all make morbid obesity second only to smoking as a preventable cause of death.

Surgical procedures for obesity date back to 1889 (Billroth) with the earliest peer reviewed procedure being the jejuno-ileal bypass in 1954 (Kreman). A successful procedure is commonly defined as one that results in at least 50% excess weight loss at 2 years. Today, the most commonly done operation is the Roux-en-Y gastric bypass (RYGB), with around 35,000 performed annually in the U.S. Other forms of bariatric surgery include Fobi pouch, bilio-pancreatic diversion, and gastroplasty or "stomach stapling". The single existing procedure that involves an implanted device is the Lap-Band, which is a laparoscopically installed inflatable cuff that is placed around the top of the stomach just below the lower esophageal sphincter (LES). This device affects satiety only (no reduced caloric absorption). Because there is more to obesity than simple overeating, it is unlikely that Lap-Band by itself will ever be as effective as a surgery that includes other physiologic feedback mechanisms.

The RYGB procedure is a procedure which has become very common in bariatric surgery. This procedure facilitates the movement of the jejunum to a high position by using a retrocolic Roux-en-Y loop. The procedure is generally performed through a 15-20 cm (6-8 inch) incision extending from the end of the breastbone to just above the navel. The stomach is completely divided into 2 unequal portions (a smaller upper and a larger lower gastric pouch) using an automatic stapling device with the raw surface reinforced with additional sutures. The upper pouch typically measures less than about 1 ounce or 20 cc, while the lower larger pouch remains generally intact and continues to secrete stomach juices flowing through the intestinal tract.

A segment of the small intestine (just distal of the duodenum or proximal of the jejunum) is then brought from the lower abdomen and joined with the upper pouch to form an end-to-end anastomosis created through a half-inch opening, also called the stoma. This segment of the small intestine is called the "Roux loop" and carries food from the upper pouch to the remainder of the intestines, where the food is digested. The remaining lower pouch and the attached segment of duodenum are then reconnected to form another anastomotic connection to the Roux loop at a location approximately 50-150 cm (1.6-4.9 ft) from the stoma, typically using a stapling instrument. It is at this connection that the digestive juices from the bypassed stomach, pancreas, and liver enter the jejunum or ileum to aid in the digesting of food. Due to the small size of the upper pouch, patients are forced to each at a slower rate and are satiated much more quickly, thereby reducing the caloric intake (typically between about 1000-1200 Calories).

Because the food enters the intestines directly, conditions know as the "dumping syndrome" are created when certain types of "junk foods" are consumed (usually sweets and other simple carbohydrates). This creates unpleasant feelings of nausea, diarrhea, nervousness, and sweating, which in turn discourages patients from developing unhealthy eating patterns. With the RYGB procedure, a loss of at least 50% of excess body weight (EBW) is maintained in approximately 60% of patients at 5 years with a reduced complication rate than other procedures.

In creating the anastomoses in the RYGB procedure, several methods have previously been developed to maintain channel integrity. However, the conventional RYGB procedure requires a great deal of operative time and because of the degree of invasiveness, post-operative recovery time can be quite lengthy and painful.

Aside from the RYGB procedure, another gastrointestinal disease which relates to the stomach is gastroesophageal reflux disease (GERD). The lower esophageal sphincter is located in a distal portion of the esophagus adjacent to the junction between the esophagus and the stomach. When food is digested, a properly functioning lower esophageal sphincter would allow food to pass from the esophagus to the stomach while preventing reverse flow. However, GERD is a disorder where the esophageal sphincter allows the stomach contents, which includes gastric acid and bile, to flow back into the distal portion of the esophagus. Some complications associated with GERD include heartburn, pulmonary disorders, chest pain, esophageal ulcers, esophagitis, Barrett's esophagus, and esophageal carcinoma.

Common treatments for GERD include the administration of prescription acid blockers. But these drugs afford only short term relief; additionally, these drugs can be expensive and may have long-term side effects. Surgical procedures have included a procedure called the Nissen fundoplication, where a portion of the gastric fundus is wrapped around the esophagus. The wrapped fundus applies pressure to the esophagus to limit the reverse flow of the stomach contents. Effectively elongating the esophagus by fundoplication or by extending it via a staple line may be done to treat GERD. Conventional fundoplication procedures may be effective at treating GERD, but they also have disadvantages. For instance, many of these procedures require large incisions to be made in a patient. Laparoscopic procedures typically require several smaller incisions formed in the abdominal wall for the insertion of instruments into the patient's body. However, such procedures can be expensive and they can increase the risks of post-operative hernias, accidental organ perforations, and other related drawbacks.

Examples related to the field of gastroplasty are describe below.

U.S. Patent No. 5,549,621 pertains to an apparatus and method for performing vertical banded gastroplasty without the use of staples. The described device uses at least two clamping bars to create a tubular-shaped pouch. However, the device is deployed laparoscopically onto the external surface of the stomach.

U.S. Patent No. 5,382,231 describes a device for transesophageal stomach retraction by a device having vacuum ports utilized to draw the stomach over the device. However, this device is used for manipulating and retracting a patient's stomach from the inside during a variety of surgical procedures and is not a permanent procedure for creating an internal pouch within the stomach itself.

U.S. Patent No. 5,345,949 relates to laparoscopic methods and tools for inserting a banding device to bring the walls of the stomach adjacent to one another between the proximal pouch and the distal region of the stomach. But there is no procedure for the creation of an internal pouch internally created from the stomach.

Examples related to the field of GERD treatment are described below.

U.S. Patent No. 6,159,146 relates to a device which is inserted transesophageally and engages the inside anterior wall of the fundus and secures it to the side of the esophagus.

U.S. Patent No. 6,113,609 pertains to a system which includes placement of a distal anchor through a hole formed in the wall of the esophagus and through a hole formed in the gastric wall, which are then fastened together.

U.S Patent No. 5,571,116 to Bolanos et al. pertains to an invagination device which approximates the lower esophagus and the fundus of the stomach.

WO-A-0078227 is considered to represent the closest prior art and discloses apparatus for GERD treatment, including a stapler capable of attaching tissue held by a vacuum gripping device.

However, all of these examples are limited to treatments for GERD which involves the attachment of the fundus, or upper portion of the stomach, to the esophagus.

According to the present invention there is provided a device for reducing the interior size of a stomach by dividing the stomach into a main pouch and a modified pouch, the device comprising a tissue adhering member having a proximal end and a distal end with a length therebetween, characterized in that:
the tissue adhering member has a slotted first vacuum port aligned parallel to a longitudinal axis of the device and for releasably adhering a first fold of stomach tissue to the slotted first vacuum port using a vacuum and a slotted second vacuum port aligned parallel to the longitudinal axis of the device and for releasably adhering a second fold of the stomach tissue to the slotted second vacuum port using a vacuum so as to enable the first and second folds of stomach tissue to be releasably positioned near to one another; and
the device further comprises a fastener disposed within the tissue adhering member, the fastener being deployable therefrom and constructed and arranged to fasten together the first and second folds of stomach tissue to form the modified pouch and the main pouch from the interior of the stomach.

Various tools and methods of treatment for obesity are described herein which are less traumatic and less invasive than procedures currently available. A variety of methods for the treatment of obesity, as well as other gastric-related diseases, e.g., gastroesophageal reflux disease (GERD), are disclosed. One method involves reducing the size of the stomach pouch to limit the caloric intake as well as to provide an earlier feeling of satiety. This may be done by creating a smaller gastric pouch within the stomach. This procedure optionally may be enhanced by performing a pyloroplasty prior to and/or in conjunction with the pouch size reduction, i.e., rendering the pyloric sphincter incompetent. This increases the rate of stomach emptying, allowing sugars and fats to pass directly into the bowel, thereby inducing dumping. Moreover, the food in the stomach may be made to also bypass a proximal portion of the bowel, i.e., a portion of the duodenum and jejunum, by creating a gastric anastomosis thereby creating a malabsorption of sugars and fats which are mostly absorbed in the bypassed portion of the duodenum and jejunum. Sugars and fats entering the bowel directly from the stomach rather than passing through the pylorus and proximal duodenum and jejunum may cause "dumping" syndrome and diarrhea. This in turn may create enforced behavioral modifications, thereby discouraging the patient from eating these types of high-caloric foods.

In forming a modified pouch, a marking device, such as a bougie, may be used at the beginning of the procedure, to create a dye marker "road map" on the interior surface of the stomach from the pylorus to the esophagus. This may enable visualization by, e.g., an endoscope, to give the physician a clear reference point for staple or fixation element placement. A distal balloon, which is preferably attached to an inflation tip at a distal end, may be inserted into the pylorus to stabilize the bougie during the procedure and may be inflated from the proximal end of the tubing by the physician.

In reducing the stomach size, one variation involves grasping the interior walls of the stomach, preferably via an endoscope advanced transesophageally, and placing one to several individual fixation elements on opposing interior walls and then bringing those fixation elements together. The stomach pouch may be modified and/or created by a variety of other device variations utilizing other methods, e.g., stapling opposing sides of a stomach together to form two separate lumens from within the interior surface of the stomach. An endoscopic stapling device may be used to accomplish such a task. Such an endoscopic stapler preferably brings two regions of tissue into apposition and may then apply a fastening element, e.g., staples, clips, tags, screws, etc., into the two regions of tissue to affix them together.

To aid in the overall effect, a pyloroplasty procedure may also be performed to enhance treatment. The pyloroplasty may be performed prior to (preferable), in conjunction with, or following the gastric reduction procedure. A pyloroplasty procedure typically results in the pyloric sphincter being rendered incompetent. Generally, a pyloroplasty device may be passed endoscopically through the esophagus, into the stomach, and preferably into position in or across the pylorus. Energy or a stimulus is then preferably applied to the pylorus to render it incompetent.

Moreover, an additional anastomosis gastric bypass procedure may also be performed to further enhance treatment. The anastomosis procedure may be performed preferably prior to, in conjunction with, or following the gastric reduction and pyloroplasty procedures (if performed at all). The procedure generally involves endoscopically or laparoscopically creating a side-to-side anastomosis preferably from within the stomach and bowel and within the digestive tract. This procedure may be similar to the Roux-en-Y gastric bypass procedure but with minimal trauma.

In using any one of the gastric reduction tools as described herein, the treatment of a hollow body organ may require multiple passes by the tools described above. Accordingly, to facilitate patient treatment, an overtube assembly may be used in conjunction with those tools. Accomplishing such treatments may require multiple passes through the esophagus by the tools used. An overtube assembly which is preferably comprised of an overtube member is disclosed which may be inserted into the hollow body organ, e.g., the stomach, through the esophagus of the patient.

The overtube may define a working lumen throughout which preferably extends from the proximal end to the distal end of the overtube. At the distal end, at least one window, and preferably two or more windows may be defined opposite of one another. The windows are preferably defined in the shape of slots near or at the distal end of the overtube. The lengths and widths of these slots are preferably long enough to approximate a desired length of a boundary or junction line within the stomach. The slots are preferably located in apposition to one another, but other variations may include offset windows separated by a dividing wall within the overtube lumen, as well as windows which are both offset and alternating located in apposition to adjacent windows. The entire length of the overtube, or at least a majority of the length, is preferably flexible enough to be inserted within the body and conform to the curvatures within the body. Alternatively, the portions of the overtube length may be made to have different regions of flexibility. The overtube may also have a bendable region having a flexibility which enables it to be manipulated or bent into arbitrary shapes either actively by the physician or surgeon or passively by an endoscopic device inserted within the overtube.

A separate drive tube may be inserted within the overtube lumen and is preferably freely adjustable, i.e., longitudinally as well as rotationally within the overtube. The drive tube itself has a lumen defined within through which an endoscopic device may be inserted within to extend beyond the distal ends of both the overtube and the drive tube to examine and/or identify tissue regions of interest. A fastener is also preferably located within the lumen of the overtube and may be located distally of the drive tube. The proximal end of the fastener may be configured to engage the drive tube and may be formed into a variety of different shapes. For instance, the fastener may be in the form of a linear shape (e.g., in the form of a spear, harpoon, rivet, etc.), in a staple configuration, or in a spiral or helical shape. The shape of the fastener is generally determined, *inter alia,* by the desired approximated tissue configuration and the configuration of the overtube, as described in further detail below. It is also preferably configured to remain attached to the drive tube or to the inner wall of the overtube lumen until the fastener is deployed into the tissue region of interest. To deploy the fastener into the tissue, the drive tube with the fastener connected thereto may be advanced distally through the overtube lumen while rotating the drive tube via a proximally actuated torquing force. As the drive tube is rotated, the fastener is advanced into the tissue while fastening the tissue in a manner similar to a screw.

The overtube assembly may also comprise a fluid port which is in fluid communication with the working lumen of the overtube and which is also in fluid communication with a pump which may be used to provide negative pressure to create a vacuum within the overtube lumen; any number of ports may be used. Furthermore, the fluid port and any other fluid ports, if used, may also be fluidly connected to positive pressure pumps either in parallel or alternatively switched. Moreover, the same pump may be used to provide both negative and positive pressure.

In use, the overtube assembly may be inserted, e.g., orally, into a patient and advanced within the esophagus until the distal end is within the stomach. Once in the stomach, the distal end may be actively or passively positioned by the physician or surgeon until the device has been desirably positioned. The pump, which is preferably in fluid communication with the overtube lumen, may then be activated to create a vacuum within the overtube to draw portions of the identified tissue within the windows. Once the tissue has been adhered and drawn into the windows, the fastener or fasteners may be advanced into the invaginated tissue to secure it. If the procedure requires additional fasteners, the overtube device may be maintained in position within the stomach while the drive tube may be withdrawn from the region to position additional fasteners. Alternatively, the drive tube may be withdrawn or maintained in position and the endoscopic device may be removed from the overtube lumen to allow for the insertion of other tools or devices through the overtube into the region.

### BRIEF DESCRIPTION OF THE DRAWINGS

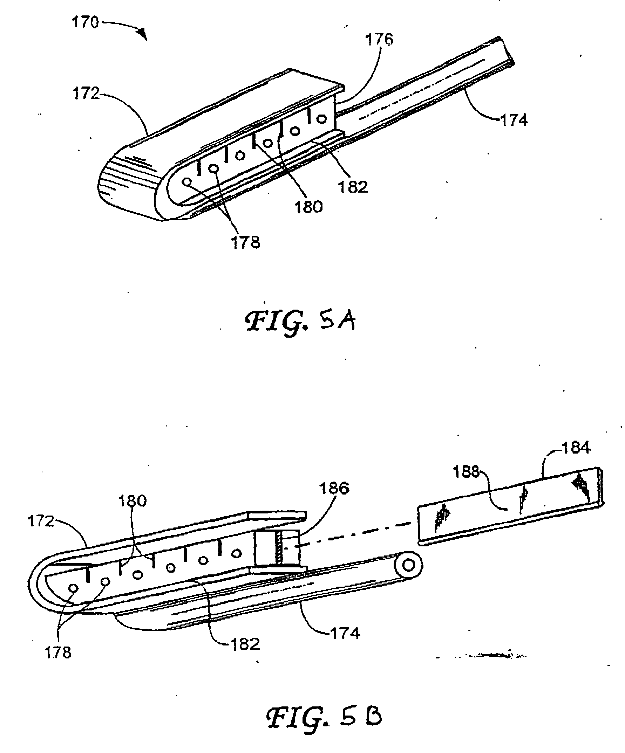

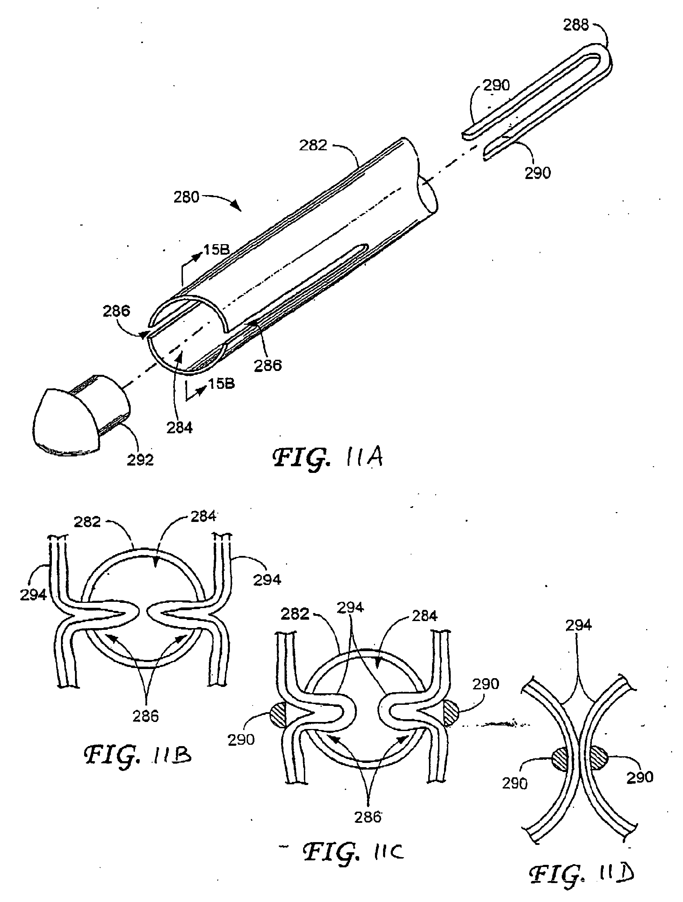

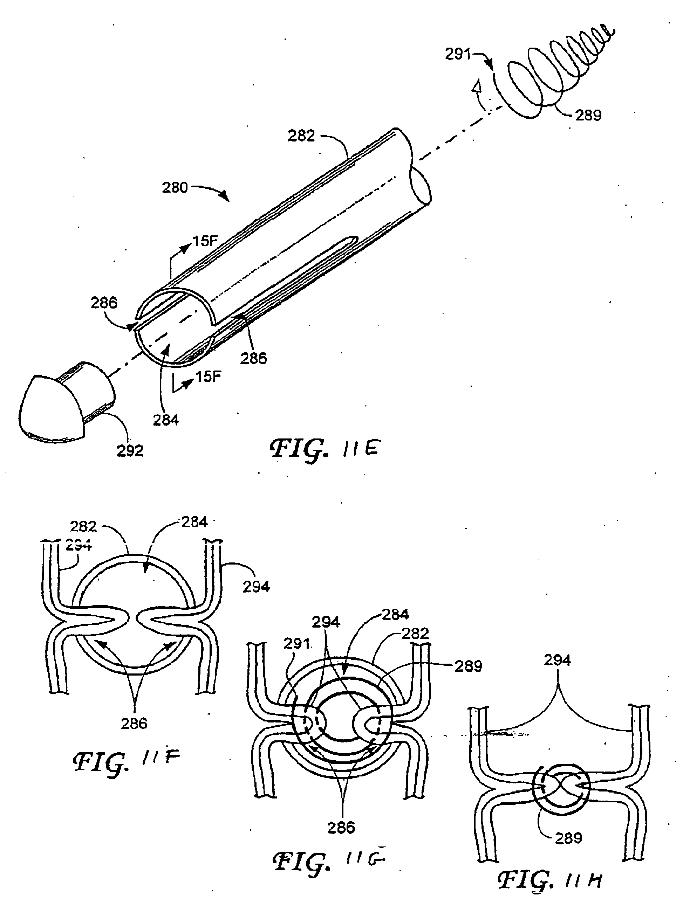

The tissue adhering members illustrated in Figs. 5-10 of the drawings are not in accordance with the present invention. Nevertheless, they will assist the reader in understanding the construction and operation of the preferred embodiments of device, which are in accordance with the present invention and which are illustrated in and described by way of reference to Figs. 11A-11D and 11E-11H.

Fig. 1A shows an example of a modified stomach having a smaller pouch created from the interior surface lining.

Fig. 1B shows a partial superior view of the cross section from Fig. 1A.

Fig. 2 shows a variation on a marking device or bougie for marking the interior surface of a stomach.

Fig, 3A shows a variation on positioning a marking device inserted into a stomach.

Fig. 3B shows a cross section view from Fig. 3A of a deflated stomach around the marking device.

Fig. 3C shows the cross section view from Fig. 3B of an insufflated stomach with the resulting marks.

Figs. 4A and 4B show a superior view of a modified stomach maintained by a fastening staple.

Figs. 5A and 5B show isometric views of a variation on an endoscopic stapling device.

Fig. 6 shows an isometric view of a variation on a box stapling device.

Fig. 7A shows an assembly view of another stapling device variation.

Fig. 7B shows a side view of the device of Fig. 7A.

Fig 8A shows an isometric view of a crescent shaped variation of a stapling device.

Fig. 8B shows an end view of the device of Fig. 8A showing a staple deploying.

Fig. 8C shows an interior side view of the device of Fig. 8A with a translating wedge sequentially deploying staples.

Fig. 9 shows an interior view of a stomach with an example of stapling device placement.

Fig. 10 shows an interior view of a stomach with an example of a modified stapling device which may be used for the treatment of GERD.

Fig. 11A shows an assembly view of a variation on an approximating device.

Figs. 11B to 11D show the process of invaginating stomach interior lining and fastening using the device of Fig. 11A.

Figs. 11E shows the assembly view of another variation of the device of Fig. 11A wherein the clip may be replaced by a screw.

Figs. 11F to 11H show the process of invaginating stomach interior lining and fastening using the device of Fig. 11E.

Fig. 12A shows an isometric view of an overtube assembly having a bendable distal region.

Fig. 12B shows optional shaping mandrels which may be inserted through or along the overtube assembly.

Fig. 13 shows a detailed assembly view of a variation on the distal end of the overtube assembly showing a fastener and a drive tube positioned within the overtube.

Fig. 14A shows a detailed isometric view of the proximal assembly of the overtube.

Fig. 14B shows a cross-sectioned profile of the assembly of Fig. 14A.

Fig. 15 shows a schematic of one example of the overtube assembly in use within a patient.

Fig. 16 shows a cross-sectioned end view of the overtube in use within a stomach lumen.

Fig. 17 shown an isometric view of the overtube within a stomach with the stomach and overtube walls partially removed for clarity.

Figs. 18A and 18B show isometric and end views, respectively, of one variation of the overtube distal end.

Figs. 19A and 19B show isometric and end views, respectively, of another variation of the overtube distal end having offset windows.

Figs. 20A and 20B show isometric views of an overtube variation within a stomach prior to tissue fixation and after, respectively.

Figs. 21A and 21B show isometric views of another variation of the overtube distal end having alternating windows.

Figs. 22A and 22B show side views of the overtube of Figs. 21A and 21B.

Fig. 23 shows a cross-sectioned side view of the overtube of Figs. 21A and 21B.

### DETAILED DESCRIPTION OF THE INVENTION

With obesity becoming an increasing problem, various tools and methods of treatment are described herein which are less traumatic and less invasive than procedures currently available. As described in further detail below, a variety of methods for the treatment of obesity, as well as other gastric-related diseases, are disclosed. Generally, the size of the stomach pouch may be reduced to limit the caloric intake as well as to provide an earlier feeling of satiety. This may be accomplished by creating a smaller gastric pouch within the stomach by a variety of methods. This procedure optionally may be enhanced by performing a pyloroplasty prior to and/or in conjunction with the pouch size reduction, i.e., rendering the pyloric sphincter incompetent. Additionally, the food in the stomach may be made to also bypass a proximal portion of the bowel, i.e., a portion of the duodenum and jejunum, by creating a gastric anastomosis thereby creating a malabsorption of sugars and fats which are mostly absorbed in the bypassed portion of the duodenum and jejunum. Sugars and fats entering the bowel directly from the stomach rather than passing through the pylorus and proximal duodenum and jejunum may cause "dumping" syndrome and diarrhea. Moreover, rendering the pylorus incompetent may also lead to dumping syndrome partly because of the rapid gastric emptying which may occur. This in turn may create enforced behavioral modifications, thereby discouraging the patient from eating these types of high-caloric foods.

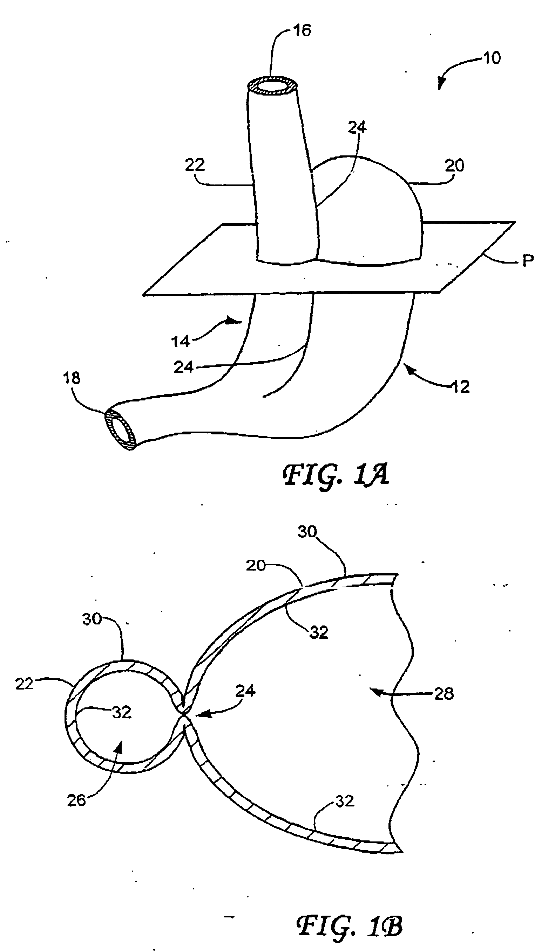

Fig. 1A shows an example of a modified stomach **10** which may be created, by any one of the methods described below, as part of the present invention. Greater curvature **12** and lesser curvature **14** is seen in modified stomach **10,** as well as the distal end of esophagus **16 and pylorus 18.** As part of the present invention, stomach **10** may be divided along junction **24** into modified pouch **22,** which is preferably less than about 28 cm³ (1 ounce) in volume, and main pouch **20.** Fig. 1B shows a partial superior view of the cross section of main pouch **20** and modified pouch **22** as viewed from cutting plane P from Fig. 1A. As seen, modified lumen **26** is preferably formed by junction **24** from main lumen **28** by joining a portion of stomach wall **30.** During ingestion of food, modified pouch **22** accepts food from esophagus **16** and preferably passes it directly through modified lumen **26** into pylorus **18.** Main pouch **20** may remain intact and function normally, but preferably sees little or no food. Acids and other fluids that may be generated in main lumen **28** may drain through the reduced outlet near pylorus **18** and may pass through the digestive system normally.

### Making Tools and Methods

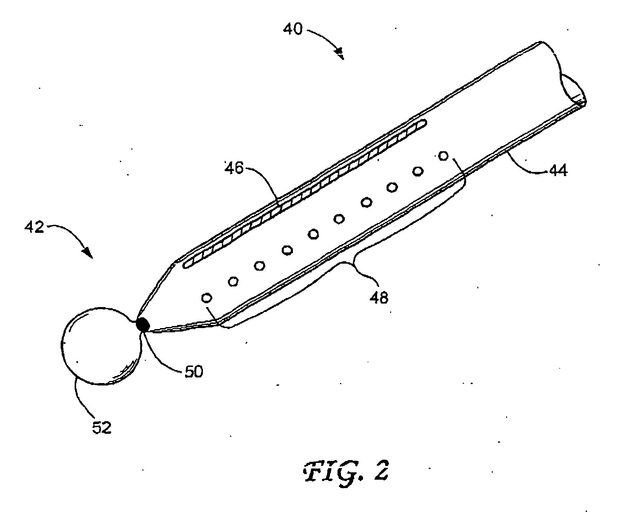

As part of forming a modified pouch, a marking device may be used, preferably at the beginning of the procedure, to create a dye marker "road map" on the interior surface of the stomach from the pylorus to the esophagus. Once such dye marks are placed, they may be visualized, e.g., endoscopically, thereby giving the physician a clear reference point for staple or fixation element placement. An example of such a marking device is shown in Fig. 2 as marking device or bougie **40.** Bougie **40** is preferably an elongated device made from tubing member **44** which may have several channels defined within. Tubing **44** may be made from any variety of biocompatible materials, e.g., stainless steel, plastics, etc., and preferably has a diameter and cross section which is similar to that of the finished modified lesser pouch. Along the length may be defined a series of dye ports **46** through which the marking dye may be channeled through from the proximal end of bougie **40.** Any variety ofbiocompatible dyes which preferably enhance visualization may be used, e.g:, methylene blue, thionine, acridine orange, acridine yellow, acriflavine, quinacrine and its derivatives, brilliant green, gentian violet, crystal violet, triphenyl methane, bis naphthalene, trypan blue, and trypan red. Also along the length and on either side of dye ports **46** may be a series of vacuum ports **48,** which are optional. A distal balloon **52,** which may be inserted into the pylorus to stabilize bougie **40** during the procedure, is preferably attached to inflation tip **50** at distal end **42** and may be inflated from the proximal end of tubing **44** by the physician.

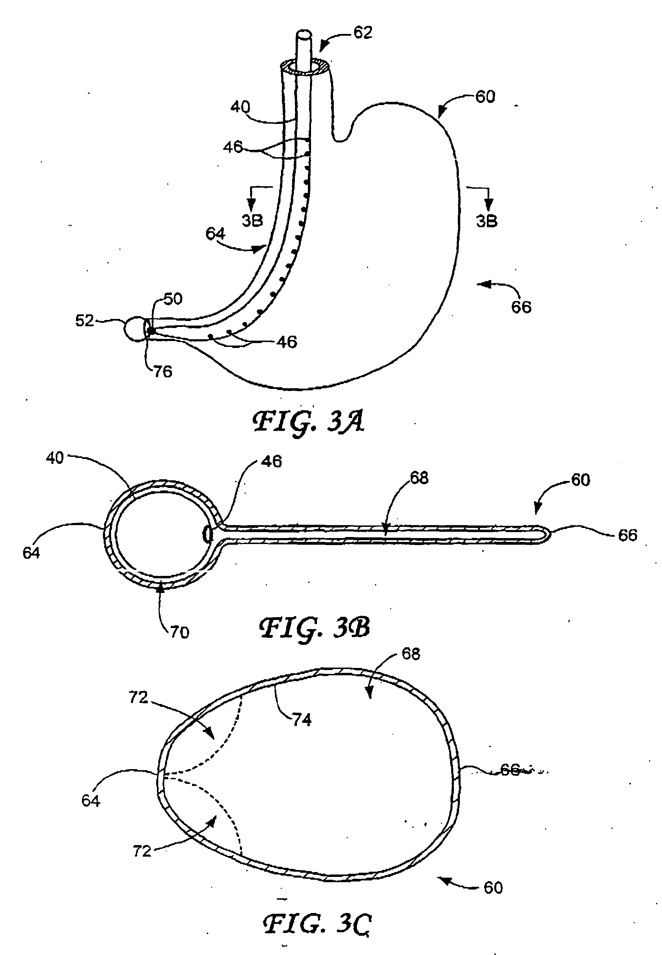

Figs. 3A to 3C show bougie **40** during one method of use. Fig. 3A shows stomach **60** as bougie **40** is inserted down through esophagus **62.** As bougie **40** is advanced down to pylorus **76,** distal balloon **52** may be inflated through inflation tip **50,** thus securing the device. Bougie **40** preferably follows lesser curvature **64** and may alternatively be shaped to approximate lesser curvature **64.** Bougie **40** is also preferably rotated such that dye ports **46** face away from lesser curvature **64** and face towards greater curvature **66.** Then the air and fluids contained within stomach **60** are preferably removed, either through vacuum ports **48,** if they are included in bougie **40,** or through another vacuum port which may be introduced endoscopically through esophagus **62.** Fig. 3B shows cross section 3B-3B from Fig. 3A as deflated stomach **60.** Once deflated, modified lumen **70** may take shape around bougie **40,** separate from deflated main lumen **68.** In this deflated state, the dye may be channeled through dye ports **46,** thereby leaving dye marks **72** on interior lining **74.** Once the staining has been performed, lumen **68** may be insufflated, as shown in Fig. 3C, and bougie **40** may then be removed. As seen in Fig. 3C, dye marks **72** mark or delineate the junction region where anchors or fasteners may be placed to draw interior lining **74** together to form the modified lumen.

### Gastric Reduction Tools and Methods Using Stapling Device

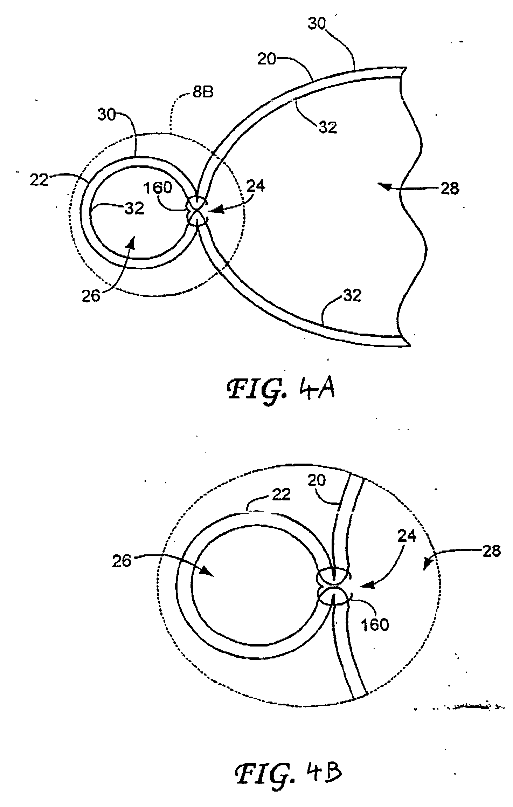

The stomach pouch may be modified and/or created by a variety of devices. Fig. 4A shows the cross sectioned superior view of Fig. 1B with the addition of staple **160** maintaining junction **24**. The figure shows an example of how, e.g., an endoscopically applied stapler, may be used to retain and hold junction **24** to form modified lumen **26.** Fig. 4B shows a close-up view of the junction **24** and staple **160** which was applied from within lumen **26.**

To staple opposing sides of a stomach together to form two separate lumens from within the interior surface of the stomach, an endoscopic stapling device may be used to accomplish such a task. Such an endoscopic stapler preferably brings two regions of tissue into apposition and may then apply a fastening element, e.g., staples, clips, tags, etc., into the two regions of tissue to affix them together. These stapling devices may optionally incorporate the use of the marking device or bougie **40,** as described above, as a preliminary step as a guide to vacuum placement and/or stapling to form the desired modified lumen. The fastening elements, e.g., staples, are preferably made of a biocompatible - material such as stainless steel, titanium, polymers, sutures, nitinol, or any other similar metals and alloys, etc. and may be in any conventional shape such as C-shaped and U-shaped staples or any of the other shapes as described herein. The two regions of tissue may be adhered to the stapling device by a variety of attachment methods, e.g., tines, barbs, hooks, vacuum, or any combinations thereof. In an adhering device utilizing a vacuum to hold the apposing regions of tissue together, such a device may be a tubular or wand-shaped member and preferably has at least two windows which may be spaced about the circumference of the tube or wand. These windows may be separated by an arc in a range of about 20° to 180° about the longitudinal axis defined by the length of the tube or wand, and are preferably separated by an arc in a range of about 90° to 180°.

Several examples of different possible variations on the stapling device are shown and described below. These variations are not intended to be limiting but are merely given as illustrative examples.

Fig. 5A shows a variation of an endoscopic stapling device in the isometric view of anvil stapling device **170.** Stapling unit **172** is shown attached to the distal end of tube **174.** Within stapling unit **172** is staple enclosure **176** where staples may be loaded and vacuum ports **178** which are seen in an alternating fashion with staple slots **180,** through which the staples may be deployed. Fig. 5B shows a reverse isometric view of the device of Fig. 5A. As seen, stapling unit **172** may have septum **184** insertable into septum slot **186,** which is preferably midway between the sides of staple enclosure **176** and which may separate the interior of staple enclosure **176** into two separate chambers. Septum **184** may serve several functions, one of which may be to allow selective activation of opposing sides of vacuum ports **178** of unit 172 as tissue is selectively adhered to the device. Other functions of septum **184** are discussed below.

In operation, stapling unit **172** may be inserted trans-esophageally into a stomach and a first portion of the interior lining may be adhered to a single side of staple enclosure **176** through a vacuum created within vacuum ports **178.** The vacuum may be created in stapling unit **172** through tube **174** and activated from the proximal end of tube **174** from outside the patient's body. Once the first portion of the interior lining is adhered to one side of staple enclosure **176,** the opposite set of vacuum ports **178** may be activated and unit **172** may be used to draw the first portion to an opposing second portion of the interior lining, which may then be adhered to the device such that the first portion and the second portion are preferably in apposition to each other. This action preferably forms the modified lumen **26** of Figs. 4A and 4B. As the tissue is held to unit **172,** septum **184** may be withdrawn from septum slot **186** by introduced forceps through, e.g., an endoscopic or through an integral actuator, to form a single chamber within staple enclosure **176.** Removal of septum **184** may then bring the first and second portions of tissue into contact apposition. The side surfaces **188** of septum **184** may incorporate a cutting, abrading, scoring, heating, freezing, chemically damaging, or some other damaging surface to tissue. Such a surface **188** may damage the interior lining contacting each other upon removal of septum **184** as surface 188 slides past. This damage may encourage a more vigorous healing response and a more permanent fixation between the damaged tissue once stapled or affixed together.

After removal of septum **184,** the staples loaded within staple enclosure **176** may be fired through staple slots **180** to affix the tissue. As the staples are fired, anvil **182** may be used as an anvil to secure the staples to the tissue, thereby resulting in the modified lumen **26** as shown in Fig. 4B. The length of stapling device **170** may be made according to the desired junction length and the size of the patient's stomach. This particular variation may be withdrawn from the area after the stapling procedure by first pushing the stapling device 170 past the resulting staple line.

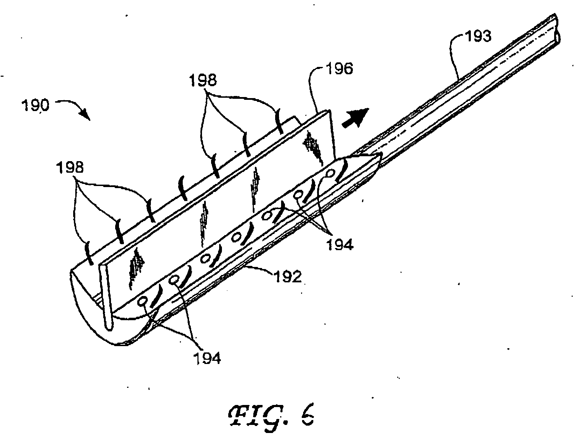

Fig. 6 shows an isometric view of another variation in box stapling device **190.** Stapling unit **192** is shown as being attached in fluid communication to vacuum tube **193.** Stapling device **190** may be inserted and operated in the same mariner as device **170** described above. Stapling unit **192** may have vacuum ports **194** activated selectively on either side of septum **196** as described above. The tips of staples **198** are shown partially deployed for illustration purposes, but are preferably not deployed until septum **196** is first retracted preferably in the direction as indicated. Septum **196** may also be configured to damage the contacting tissue upon septum **196** withdrawal in the same manner as described above. Stapling device **190** may be easily applied and removed after staples 198 have been deployed.

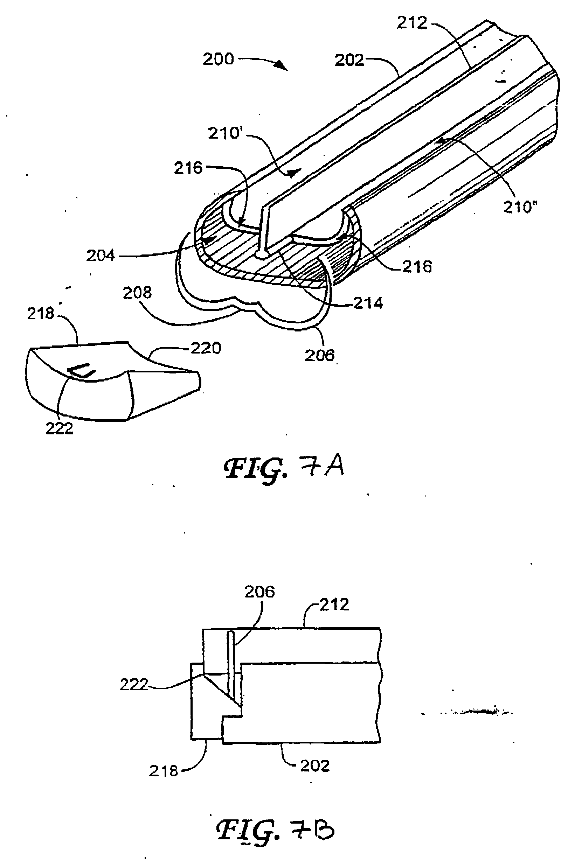

Fig. 7A shows an assembly isometric view of another variation in stapling device **200.** This variation **200** shows curved tube **202** which may have lumen **204** house staples **206** as well as act as a combination vacuum and staple slot **216.** Tube **202** may be shaped in a variety of ways but is shown here as a C-shaped or U-shaped tube with first channel **210'** and second channel **210"** for adhering the two apposed portions of tissue, preferably separated by removable septum **212.** With this variation **200,** tissue may be adhered within the channels **210', 210"** through vacuum/staple slot **216** and once positioned, staples **206** may be deployed while septum **212** is removed simultaneously by the use of curved wedge **218.** In operation, curved wedge **218** maybe drawn within lumen **204** from the tube **202** distal end to the proximal end by, e.g., a pull-wire attached to wedge **218.** As wedge **218** is advanced proximally, wedge **218** would preferably force pivot **208** of staple **206** against contact edge **214** of septum **212.** As wedge **218** is advanced further proximally, urging end **220** may then urge the curved ends of staple **206** to rotate about pivot **208** and deploy through slot **216.** While staple **206** is deploying, notch **222,** preferably located at a distal end of wedge 218, may engage contact edge **214** and begin to slide septum **212** simultaneously towards the proximal end of tube **202.** Fig. 11B shows a side view of stapling device **200** of Fig. 7A. As seen, curved wedge **218** preferably contacts septum **212** via notch **222** and pushes while simultaneously urging staple **206** to deploy. The figures show a single staple **206** for illustrative purposes only and any plurality of staples **206** may be used in practice depending upon the desired results.

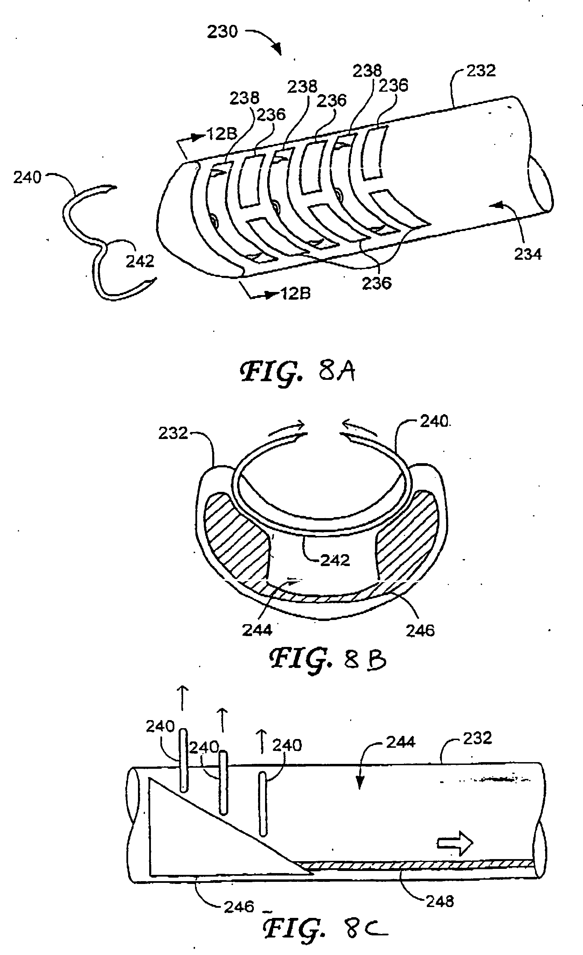

Fig. 8A shows an isometric view of yet another variation in stapling device **230.** This variation may omit a removable septum. Curved tube **232** is preferably curved in this variation in a crescent shape forming contact channel **234.** Within contact channel **234,** a number of vacuum ports **236** and staple slots **238** may be defined in an alternating pattern, as shown. A possible W-shaped staple **240** preferably having pivot **242** at the staple **240** midpoint is shown outside of tube **232** for illustrative purposes in a possible orientation for insertion within staple slots 238. Fig. 8B shows cross section 8B-8B from Fig. 8A. As seen, tube **232** defines lumen **244,** which preferably runs the length of tube **232,** and translating wedge **246** which is preferably slidingly disposed within lumen **244.** As seen in Figs. 8B and 8C, which is a side view of the interior of tube **232,** wedge **246** may be translated by pull-wire **248.** Pull-wire **248,** which may be made of any high-strength material, e.g., stainless steel, nitinol, nylon, polymers, etc., may be manipulated by a physician from the proximal end of tube **232** from outside of the patient's body. Like the device **200** of Figs. 7A and 7B, once vacuum ports **236** have acquired the interior tissue lining to be approximated, translating wedge **246** may be advanced proximally. Advancing wedge **246** may urge staples **240** to deploy through staple slots **238** sequentially as shown to hold the tissue and form the desired lumen.

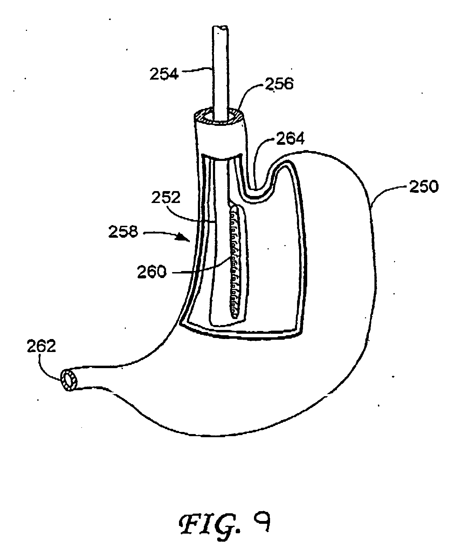

An example of deployment for any of the stapling devices described above is shown in Fig. 9. As shown, stomach **250** with the wall partially cut out is seen with stapling device **252** inserted within. Stapling device **252** is shown merely as an example of insertion and could comprise any of the devices described herein. Device **252,** which is preferably advanced trans-orally into stomach **250** and through esophagus **256,** is preferably located at the distal end of delivery/vacuum tube **254.** Once inserted, device **252** may be located by the assistance of the lesser curvature **258** of stomach **250.** Also shown are vacuum/staple ports **260,** which may be any of the configurations as described herein. In a preferable variation, stapling device **252** may be configured to produce a staple line or junction following the lesser curvature beginning from cardiac notch **264** down towards pylorus **262.** Accordingly, device **252** may have the length and vacuum/staple ports **260** configured such that the distal end of device **252** points towards pylorus **262.**

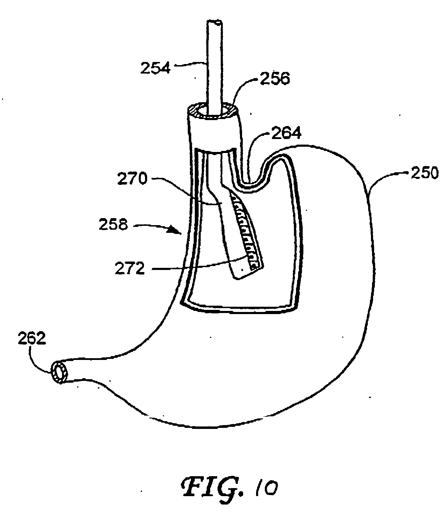

Fig. 10 shows stapling device **270** in a slightly different configuration for the treatment of other gastro-intestinal diseases such as gastroesophageal reflux disease (GERD), as discussed above. The stomach **250** of Fig. 9 is shown, but for the treatment of GERD, stapling device **270** may be slightly modified such that the device **270** and vacuum/staple ports **272** may be straight or flared away from, rather than towards, lesser curvature **258** and pylorus **262** as described above. As such, vacuum/staple ports **272** would preferably produce a staple line or junction beginning from cardiac notch **264** and then flares away from lesser curvature **258** and pylorus **262.** Device **270** may be any of the devices described and operated herein, but for the flared modification. Likewise, any of the devices described herein may be used for the treatment of GERD by simply angling the device to produce a flared staple line. Alternatively, a simple non-flared staple line may also suffice for treating GERD. The staple line may act as a Heimlich valve which preferably closes down in response to pressure exerted from the greater or main lumen. Moreover, the smaller volume of the modified lumen in-line with esophagus **256** may provide a smaller volume of acid available for esophageal reflux.

An isometric view of a single channel vacuum device variation is shown in Fig. 11A in approximating device **280**. Tube **282** is preferably a tubular device which may be inserted into a stomach through the esophagus of a patient. A lumen **284** may run through tube **282** from a proximal end to the distal end of tube **282.** At the distal end, two or more windows or slots **286** are preferably defined opposite of one another, as shown. The lengths and widths of slots **286** may vary and is preferably long enough to approximate the desired length of the boundary or junction line of the modified lumen; likewise, the width is preferably wide enough to accommodate at least two layers of the stomach interior lining. Approximating clip **288** is shown having at least two piercing ends **290** and may be loaded into tube lumen **284** from either the proximal end or distal end of tube **282** preferably prior to inserting the device **280** into the patient. Clip **288** is preferably made of a biocompatible material as described above. Biodegradable plug **292** may be placed into the distal end of tube **282** prior to insertion into the patient and is preferably made of a biocompatible biodegradable material, e.g., biodegradable polymers such as polylactide, polyglycolide, and their copolymers. Plug **292** may be alternatively made from a non-biodegradable material and may simply pass after the procedure. Plug **292** may aid in maintaining a vacuum seal through slots **286** during the approximation procedure, as described below.

Fig. 11B shows an end view from section 11B-11B from Fig. 11A of tube **282** in operation. As shown, opposing portions of stomach interior lining **294** may be drawn into lumen **284** through opposing slots **286** by creating a vacuum within lumen **284.** Approximating clip **288** may be urged distally through tube **282** such that each of ends **290** may be drawn through a corresponding slot **286** over and/or pierced through lining **294** within lumen **284.** As lining **294** is approximated within lumen **284,** biodegradable plug **292** may become invaginated within lining **294.** Accordingly, as clip **288** and ends **290** are positioned over lining **294,** tube **282** may be withdrawn from the area while clip **288** preferably slides through the distal end of tube **282** leaving the approximated interior lining **294** held in position by ends **290,** as seen in Fig. 11D. Removal of tube **282** may urge plug **292** to slide off the distal end of tube **282** and remain within the newly formed lumen to become degraded over time or to pass through the patient's system.

Fig. 11E shows the device of Fig. 11A, but in this variation, clip **288** may be replaced by coil **289,** which is preferably in the shape of a helix or a spiral having a tapering width or diameter. The first few turns of coil **289** may have the same or similar diameter than the remaining tapering turns; this may enable piercing end **291 to** engage interior **294** and may also allow coil **289** to be advanced at the desired orientation through the tissue. Coil **289** preferably maintains a parallel orientation with tube **282** during delivery into the tissue, i.e., a longitudinal axis defined by coil **289** is preferably parallel, or close to parallel, with the longitudinal axis defined by tube **282.** Moreover, the outer diameter of the first few turns are preferably the same diameter, or slightly less than, the inner diameter of tube **282.** This may further enable coil **289** to be advanced through lumen **284** at the proper orientation prior to engaging interior **294.**

As described above for the device of Figs. 11A to 11D, opposing portions of stomach interior lining **294** may be drawn into lumen **284** through opposing slots **286** by creating a vacuum within lumen **284,** as shown in Fig. 11F. Coil **289** may then be urged through lumen **284** and rotated in the direction of the arrow shown until piercing end **291** engages the invaginated lining **294.** Piercing end **291** preferably is sharp and needle-like to enable piercing through multiple layers of lining **294.** As coil **289** is further rotated, it may be further advanced distally through the remaining portion of invaginated lining **294.** The tapering diameter and decreasing width may also begin to further approximate the opposing edges of lining **294** towards one another, as shown in Fig. 11G. Finally, as seen in Fig. 11H, further advancement of coil **289** preferably draws the opposing surfaces into contact with one another. Tube **282** may then be removed, as described above. Although the fixation of one coil **289 is** described, multiple coils **289** may be fastened one after another to form a continuous fixation line.

Coil **289** may be made of a bioabsorbable or biocompatible material, as described herein such as a polymer or superelastic alloy, and may be integrally formed with barbs or whisker-like filaments protruding along its length to help prevent coil **289** from backing out once it has been engaged within the lining **294.** An example of a spiraling suturing needle or screw which may be used in this variation is shown and described in U.S. Patent No. 5,330,503. Another example of a helical fastener or screw and applicator which may be used in this or another variation is shown and described in U.S. Patent No. 5,582,616. Other examples of of helical fasteners or screws and applicators are also shown in U.S. Patent No. 5,810,882; U.S. Patent No. 5,824,008; and U.S. Patent No. 5,964,772.

### Gastric Reduction Tool Overtube System

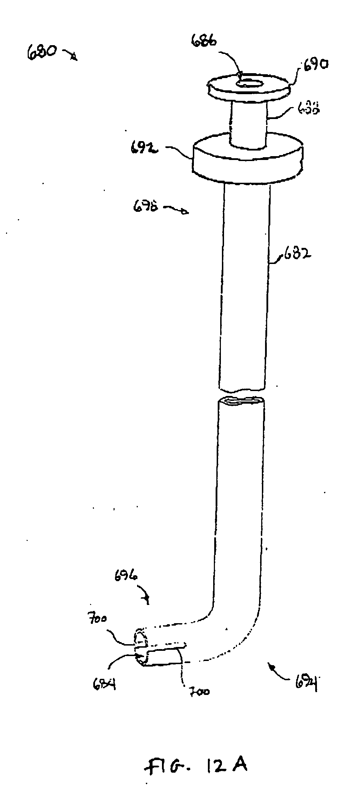

In using any one of the gastric reduction tools as described above, e.g., those as shown in Figs. 5A-10, the treatment of a hollow body organ may require multiple passes by the tools used. Accordingly, to facilitate patient treatment, an overtube assembly may be used in conjunction with those tools. Fig. 12A shows an isometric view of overtube assembly **680** which can be used to efficiently affect treatment with minimal discomfort to a patient Overtube assembly **680** is preferably comprised of overtube member **682** which may be inserted into the hollow body organ, e.g., the stomach, through the esophagus of the patient. A working lumen **684** may be defined through overtube **682** from proximal end **698** at overtube module **692** to distal end **696** of overtube **682.** At distal end **696,** at least one window **700,** and preferably two or more windows **700** are preferably defined opposite of one another in this variation, as shown. Windows **700** are preferably defined in the shape of slots near or at the distal end **696** of overtube **682.** The lengths and widths of slots **700** may vary and are preferably long enough to approximate a desired length of a boundary or junction line within the stomach, as discussed below. The stomach is comprised of at least three layers including the inner mucosal layer (mucosa), a muscular layer exterior to the mucosa (muscularis mucosae), and an exterior serosal layer (serosa). The widths of window **700** are preferably wide enough to accommodate at least two layers of the stomach interior lining and more preferably all the layers of the stomach. Illustrative widths of window **700** may range anywhere from about 0.320 cm (0.125 in.) to 0.950 cm (0.375 in.) and may range anywhere in length from about 0.635 cm (0.250 in.) to 15.25 cm (6 in.). Overtube assembly **680,** including the variations described herein, may be used to effect a variety of treatments in combination with the variety of tools and methods as described above.

Several aspects of the present invention were arrived at after experimentation with stomach tissue and the challenges of acquiring and securing such tissue reliably. In particular, it is desirable that the assembly **680** consistently approximate the tissue such that when fasteners are delivered, as described herein, they consistently reach the outer layers, or fibrous layers, of the stomach wall, such as the muscularis and serosa. The present invention may assist in this by acquiring tissue such that these fibrous layers intersect or overlap within working lumen **684,** allowing them to be secured together. Once these fibrous layers are fastened appropriately, they will adhere, fuse, or scar over to affect the desired fastening. The tissue is preferably maintained in apposition with, e.g., the two folds, for 2-4 weeks to affect healing, but that fusion of the tissue may take place as soon as 5-10 days following a procedure. If tissue folds are fastened inconsistently, or if the fasteners only penetrates the less fibrous tissue, such as the mucosa, complications such as gastric erosion, ulceration, and failure of the secured walls may result.

The entire length of overtube **682,** or at least a majority of the length of the device, is preferably flexible enough to be inserted within the body through, e.g., the esophagus, and conform at least partially to the curvatures within the body. For example, overtube **682** may range in length from about 40 cm (15.75 in.) to 100 cm (39.40 in.), and is preferably about 80 cm (31.50 in.). The overall diameter of overtube **682** may range from about 5 mm (0.20 in.) to 30 mm (1-18 in.), and preferably from about 15 mm (0.60 in.) to 17 mm (0.70 in.). Alternatively, the length of overtube **682** may be sufficiently flexible yet define a bendable region 694 near distal end **696** which may be more flexible than the rest of overtube **682.** Bendable region **694** may have a flexibility such that it may be manipulated or bent in arbitrary shapes either actively by the physician or surgeon or passively by an endoscopic device inserted within overtube **682**. Examples of tubular structures which are actively manipulatable and selectively rigidizable may include those found in U.S. Patent Nos. 5,624,381 (Kieturakis) and 4,790,294 (Allred, III et al.), among others.

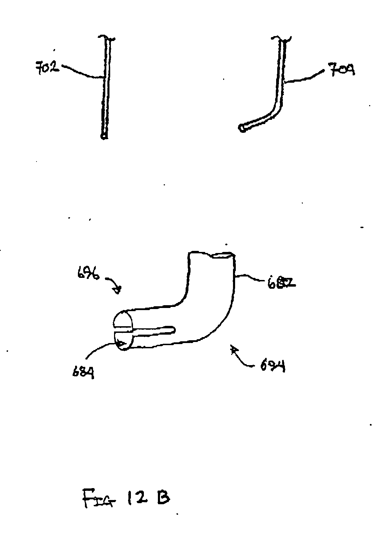

If passively manipulatable, overtube **682** may be made from any variety of biocompatible materials which provide sufficient structure to the device yet allows for the device to be bent, e.g., thermoset or thermoplastic materials such as PTFE, FEP, polyurethane, PVC, silicone, Nylon, pellethane, etc. Alternatively, overtube **682** may be preformed to have a bent or arcuate configuration near or at distal end **696.** When inserted into a patient, a straightening mandrel **702** or endoscopic device may be positioned within the curved region **694** to maintain a straightened configuration, as shown in Fig. 12B. When overtube **682** has been desirably positioned within the patient, the straightening mandrel **702** may be withdrawn from the device leaving the overtube **682** to reconfigure itself to its preformed shape. Another alternative is to have overtube **682** remain passively manipulatable and to have the insertable mandrel preformed into the curved configuration **704,** as also seen in Fig. 12B. In this case, the overtube **682** may be inserted in its straightened configuration and the curved mandrel **704** may be inserted through the overtube **682** in a constrained and straightened configuration through overtube **682.** When curved mandrel **704** reaches the distal end **696,** mandrel **704** may be allowed to curve into its preformed shape which may force overtube **682** to reconfigure with the mandrel **704**. The mandrel **704,** in this case, is preferably made of a shape memory alloy, e.g., a nickel-titanium alloy such as Nitinol. In either case, the mandrel may be inserted within an internal working channel of overtube **682** or along a exterior channel defined along the outer surface of overtube **682.**

Overtube **682** may also be made to have a proximal portion which is relatively stiffer than bendable region **694** or distal end **696**. Also, overtube **682** may have a wall thickness which depends upon the type of material used for construction which is sufficient to maintain the structural integrity of the device during use. For example, a wall thickness ranging from about 0.80 mm (0.032 in.) to 6.35 mm (0.250 in.) may be used.

A separate drive tube **688** is preferably inserted within lumen **684** of overtube **682** through overtube module **692.** Drive tube **688** is preferably a tubular member which has an outer diameter which is smaller than the inner diameter of overtube **682.** Drive tube **688** may be fabricated from the same or a similar material as overtube **682** or any of the other materials described above. There is preferably enough clearance between tubes **682, 688** such that drive tube **688** is freely adjustable within overtube **682,** i.e., freely movable longitudinally and/or rotationally within overtube **682**. Drive tube **688** is made to have a lumen defined through the tube **688** from a proximally located insertion port **686** to the distal end of tube **688.** To prevent drive tube **688** from being pushed entirely through overtube **682,** drive tube stop **690** may be located at the proximal end of tube **688.** Stop **690** may be any projection, e.g., an enlarged diameter disk, which abuts against overtube module **692** to prevent the further advancement of drive tube **688** within overtube **682.** Stop **690** may be made from a material similar to tube **688** or it may be made from a biocompatible metallic material such as stainless steel, platinum, etc., and attached to drive tube **688.**

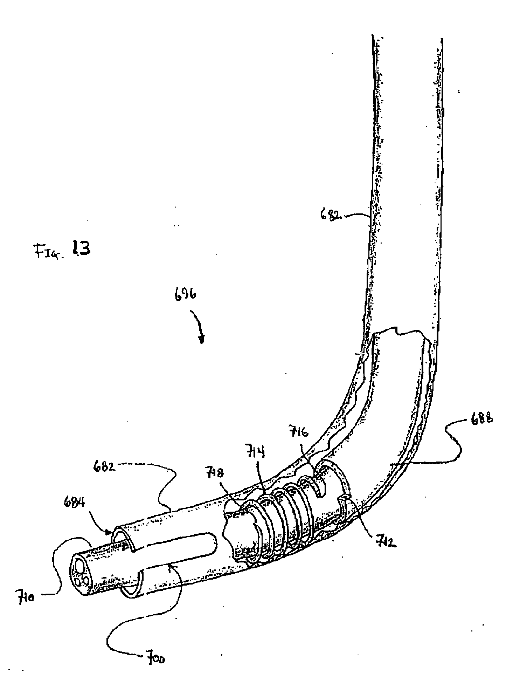

A detailed assembly view of the distal end **696** of overtube assembly **680** is shown in Fig. 13 with the wall of overtube **682** partially removed for clarity. As shown, overtube **682** may have drive tube **688** inserted within lumen **684** and extending towards the distal end of overtube **682.** An endoscope **710**, which may comprise any conventional endoscopic device preferably having optical viewing capabilities (e.g., through optical fibers), may be inserted within drive tube **688** at its proximal end through insertion port **686**. Endoscope **710** may be advanced distally through both overtube **682** and drive tube **688** to extend beyond the distal end **696** of overtube **682** to examine and/or identify tissue regions of interest.

Also preferably located within lumen **684** near or within the distal end 696 of overtube **682** is fastener **714.** Fastener **714** is preferably located distally of drive tube 688 and may be configured to have a proximal end **716** for engagement with fastener engagement area **712** of drive tube 688. It may be further configured to have a tapered distal tip **718** for piercing tissue as fastener **714** is advanced distally. As shown, fastener **714** may be configured in a spiral or helical shape having at least two revolutions and it may be configured to form an inner diameter which is large enough to allow endoscope **710** to pass therethrough uninhibited. During advancement of overtube **682** into the body or during examination of the tissue with endoscope **710,** fastener **714** may be maintained within lumen **684** by some temporary attachment device against the inner wall of lumen **684.** Preferably, however, fastener **714** may be attached via proximal end **716** to area **712** until fastener **714** is deployed into the tissue. Proximal end **716** may be temporarily connected to area **712** via a conventional mechanical engagement, such as a friction fit or a détente located within area **712,** or via an electrically-actuated joint or connection, e.g., an electrically-erodable joint.

Once fastener **714** is to be deployed into the tissue, drive tube **688** with fastener **714** connected thereto, may be advanced distally through lumen **684** and drive tube **688** may then be rotated via a proximally actuated torquing force. The torquing force may be actuated either manually or automatically with a motorized assembly (not shown). As drive tube **688** rotates about its longitudinal axis, fastener **714** also rotates and advances into the tissue region of interest while fastening the tissue in a manner similar to a screw.

Fastener **714,** which is preferably in the shape of a helix or spiral, may optionally have a tapering width or diameter. The first few turns or coils of fastener **714** may have the same or similar diameter than the remaining tapering coils; this may enable distal piercing end **718** to engage the tissue and may also allow fastener **714** to be advanced at the desired orientation through the tissue. Fastener **714** preferably maintains a parallel orientation with overtube **682** during delivery into the tissue, i.e., a longitudinal axis defined by fastener **714** is preferably parallel, or close to parallel, with the longitudinal axis defined by overtube **682.** Moreover, the outer diameter of the first few turns or coils may be the same diameter, or slightly less than, the inner diameter of overtube **682.** This may further enable fastener **714** to be advanced through lumen **684** at the proper orientation prior to engaging the tissue.

Fastener **714** may be made of a bioabsorbable or biocompatble material, as described herein such as a polymer or superelastic alloy, or a metal, e.g., stainless steel, platinum, titanium, etc., and may be integrally formed with barbs or whisker-like filaments protruding along its length to help prevent fastener **714** from backing out once it has been engaged within the tissue. Fastener **714** may be the same or a similar fastener as screw **289** described above. It may also be similar to the spiraling suturing needle or fastener shown and described in U.S. Patent No. 5,330,503.

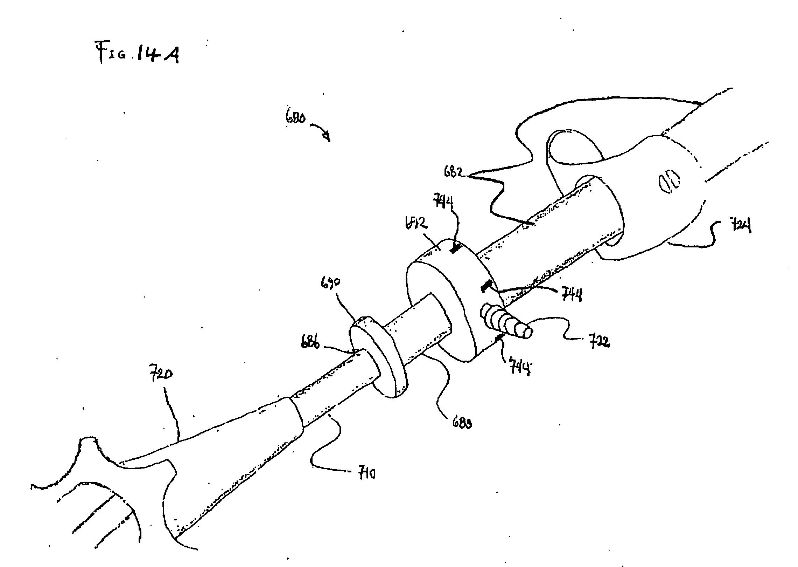

Fig. 14A shows a detailed isometric view of the proximal assembly of overtube assembly **680** with endoscope **710** inserted within insertion port **686.** The shaft of endoscope **710** may be seen almost fully inserted within assembly **680** up to endoscope handle **720** through insertion port **686** located in drive tube stop **690**. Endoscope **710** may be selectively advanced and withdrawn by the physician or surgeon into assembly **680** or it may be withdrawn completely from insertion port **686** during a procedure to allow for the insertion of other tools or devices.

Also seen extending from overtube module **692** is fluid port **722** which may be included as part of overtube assembly **680.** Fluid port **722** is a port which is in fluid communication with working lumen **684** defined within overtube **682** and may be in fluid communication through, e.g., a hose, to a pump which may provide the negative pressure to create a vacuum within lumen **684.** Although a single fluid port **722** is shown, any number of ports may be used. If multiple ports are utilized, each port may be fluidly connected to the same or a different pump. Furthermore, fluid port **722** and any of the other fluid ports, if used, may also be fluidly connected to positive pressure pumps either in parallel or alternatively switched. Alternatively, the same pump may be used to provide both negative and positive pressure. Positive pressure pumps may be used to provide the adequate pressure to deliver fluids through fluid port **722** into lumen **684** for delivery of, e.g., therapeutic drugs, saline, or gases such as nitrogen for insufflating an area within the body, among other fluids.

Any number of markers **744** may be placed upon the overtube assembly **680**, e.g., upon overtube module **692** as shown in Fig. 14A. Markers **744** may be placed upon module **692** to correspond with the circumferential location of windows **700** and may thus be used as a guide for aligning windows **700** within the patient. Overtube **682** may be manipulated and rotated from outside the body to align markers **744** with a landmark located on the patient, e.g, alignment with the patient's nose. In this manner, windows **700** of overtube **682** may be desirably positioned within the patient through external manipulation without the need to align windows **700** by direct visualization within the patient.

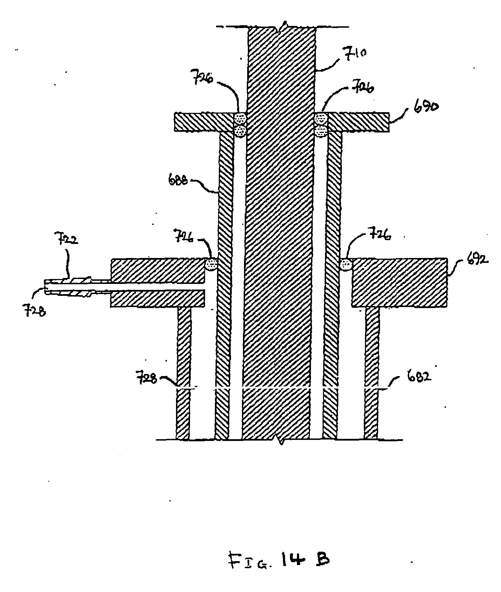

As drive tube **688** may be selectively advanced and withdrawn relative to overtube **682** and also relative to endoscope **710**, a fluid-tight seal is preferably maintained between overtube module **692** and drive tube **688.** This fluid-tight seal is preferably sustained in order to maintain the pressure (positive or negative pressure) within lumen **684** through fluid port **722,** and more specifically within the communication lumpen **728** which is created between the outer surface of drive tube **688** and the inner surface of overtube **682,** as seen in Fig. 14B, which shows a cross-sectioned profile of the assembly of Fig. 14A and may be accomplished by any number of conventional sealing methods. For instance, overtube module **692** may be gasketed **726** against the outer surface of drive tube **688** to maintain the seal between the two, as also seen in Fig. 14B. Alternatively, other types of seals using, e.g., sealing gels, ferrofluidic seals, etc., may be utilized. In addition to maintaining fluid-tight seals, guard **724** may also be included in assembly **680** to provide structural strength to the area of tissue through which assembly **680** is inserted and also to prevent overtube **682** from being pinched or crushed. For instance, if assembly **680** were inserted orally into the esophagus of a patient, guard **724** may be inserted into the mouth of the patient and used as a bite guard to prevent the patient from biting down on overtube **682.** Also, guard **724** would also ensure a smooth pathway for overtube **682** through the mouth of the patient.

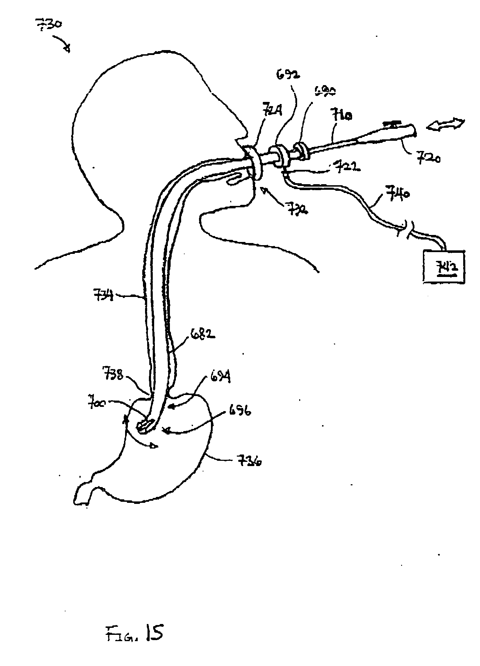

Fig. 15 shows a schematic of one example of assembly **680** in use within a patient **730**. As shown, assembly **680** may be inserted orally through mouth **732** of patient **730** and advanced within esophagus **734** until distal end **696** is advanced beyond gastro-esophageal junction **738** and into stomach **736.** Once in stomach **736,** distal end **696** may be actively or passively positioned by the physician or surgeon about bendable region **694** until the device has been desirably positioned. Then pump **742,** which is preferably in fluid communication with communication lumen **728** within overtube **682** through fluid tube **740,** may be activated to create a vacuum within overtube **682** to draw portions of the identified tissue within windows **700.** A vacuum force of about 15 to 20 inch-Hg may be utilized, although the amount of vacuum pressure may be varied depending upon the size of the windows **700** and the amount of tissue to be drawn into overtube **682.** As mentioned above, pump **742** may also be used as a positive pressure pump which may then be used to deliver therapeutic agents, fluids, or gases through tube **740** and overtube **682** for dispersion within stomach **736**.

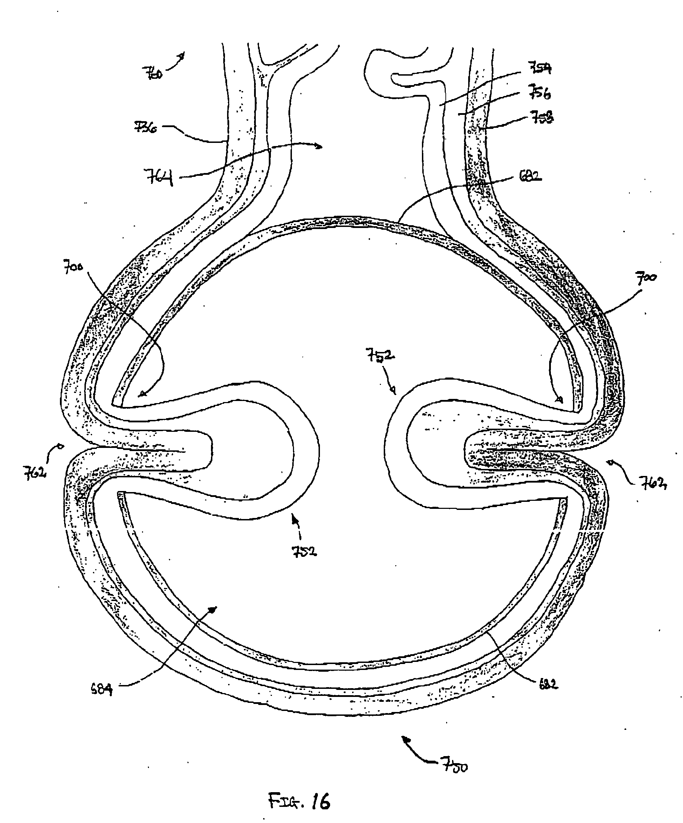

Fig. 16 shows a cross-sectioned end view of overtube **682** within stomach lumen **764** of stomach **736** once the vacuum has been created within working lumen **684.** As shown, overtube **682** may be positioned adjacent relative to lesser curvature **750** of stomach **736;** however, overtube **682** may alternatively be positioned closer to greater curvature **760** or anywhere inbetween depending upon the desired treatment and effects upon the patient. For instance, overtube **682** is preferably positioned closer to lesser curvature **750** prior to approximating the walls of stomach tissue such that a modified lumen may be created by overtube assembly **680** leading directly from esophagus **734** to the pyloral sphincter of stomach **736.** Alternatively, overtube **682** may be positioned closer to greater curvature **760** for the treatment of GERDs. As seen, overtube **682** may acquire the lining of the stomach and draw several layers of stomach tissue, i.e., mucosal layer **754,** muscular layer **756**, and serosal layer **758,** into lumen **684** through windows **700** while creating creases **762** within the outer surface of stomach **736** which may denote where stomach **736** has been invaginated. All the layers of stomach **736** need not be invaginated, but at least two layers are preferably drawn in, and more preferably all the layers, so that adequate tissue exists within lumen **684** for approximating the walls of stomach **736.** The vacuum is preferably maintained until the invaginated tissue **752** is drawn within lumen **684** such that a fastener, as described further below, may grasp both, or several, regions of invaginated tissue **752** and approximate or maintain the tissue.

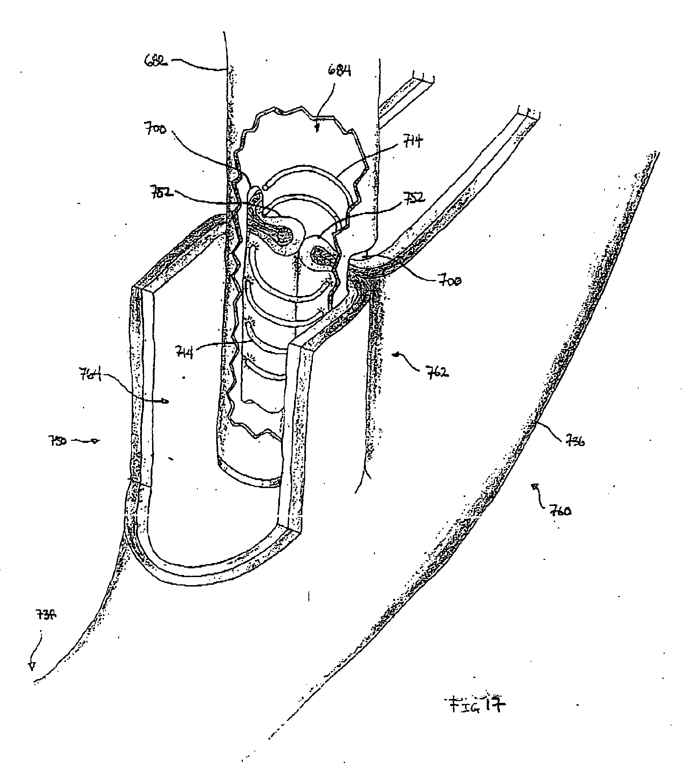

Fig. 17 shows an isometric view of overtube **682** within stomach **736** with the stomach and overtube walls partially removed for clarity. As shown, overtube **682** may be inserted, as described above. Once desirably positioned within stomach lumen **764,** a vacuum may be created within working lumen **684** of overtube **682** and tissue **752** may become invaginated within lumen **684** through windows **700,** as shown. Once the invaginated tissue **752** has been adequately drawn into overtube **682,** any number of flexible endoscopic stapling devices, as described herein, may be used to hold the suctioned tissue together.

Alternatively, fastener **714** may be advanced distally while rotating it using drive tube **688,** which is shown as having been withdrawn. As fastener **714** is rotatingly advanced, its piercing distal tip **718** may wind into and around invaginated tissue **752** in an alternating helical or spiral pattern until fastener **714** has been fully advanced into tissue **752.** Fastener **714** may be configured to have an inward bias such that once fastener **714** has been deployed within tissue **752,** the coils of fastener **714** bias inwardly such that the fastener **714** diameter shrinks and fastener **714** elongates. A single fastener **714** may be used to bind the apposing walls of tissue **752** within stomach **736,** in which case fastener **714** is preferably an elongated fastener sufficiently long enough to bind the tissue. Alternatively, multiple fasteners **714** may be fastened one after another to form a continuous fixation line. In which case overtube **682** may be advanced to a distal position within stomach **736** and a portion of the tissue walls may be fastened, as described above. Overtube **682** may then be pulled proximally a short distance within stomach lumen **764** and repositioned.

While maintaining the position of overtube **682** within stomach lumen **764,** drive tube **688** may be withdrawn from overtube lumen **684** and another fastener **714** may be inserted within overtube **682** and advanced distally to fasten another portion of the tissue wall. This process may be repeated as necessary until the desired length or locations of tissue have been fastened. An alternative configuration for the drive tube may include a clamping device for holding the approximated tissue to one another while fastening the tissue together, as discussed in further detail below.

A biodegradable plug, such as plug **292** described above, may optionally be placed into the distal end of overtube **682,** also in much the same manner as above, prior to insertion into the patient and is preferably made of a biocompatible biodegradable material, e.g., biodegradable polymers such as polylactide, polyglycolide, and their copolymers. The plug may be alternatively made from a non-biodegradable material and may simply pass after the procedure. The plug may aid in maintaining a vacuum seal through windows **700** during the fastening procedure.

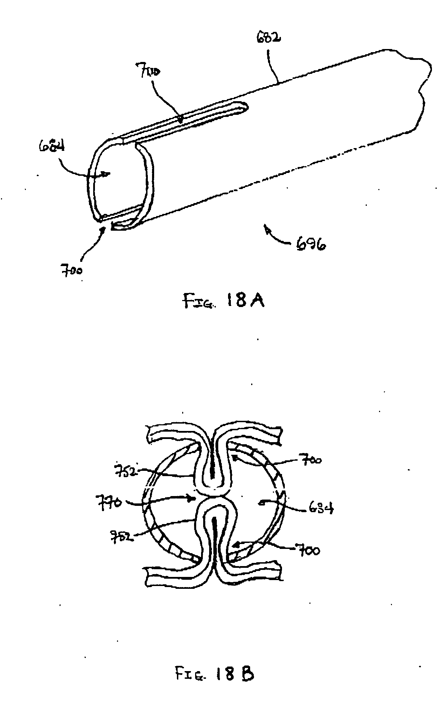

Figs. 18A and 18B show detailed isometric and end views of one variation of distal end **696** of overtube **682.** As shown in Fig. 18A, windows **700** may be formed into slots, as also described above. Fig. 46B shows an end view with invaginated tissue **752** projecting into lumen **684.** The apposed portions of tissue **752** may be drawn into overtube **682** via a vacuum force until the tissue **752** portions contact one another at contact area **770.**

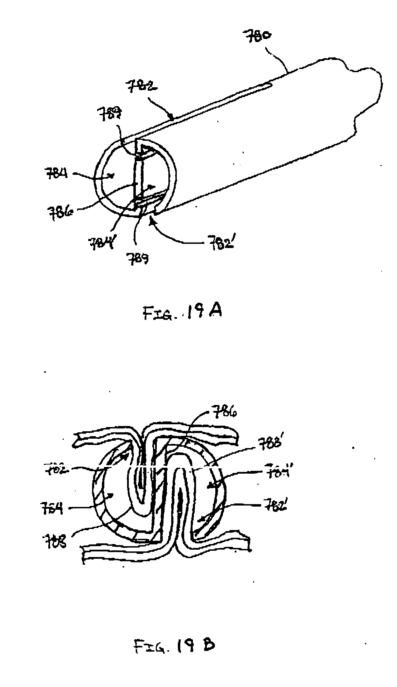

Another variation for configuring the distal end of the overtube is shown in Figs. 19A and 19B. As seen in Fig. 19A, an isometric view of overtube offset variation **780** may have the apposed windows **782, 782'** defined along the length of overtube **780** near or at the distal end, as described above. However, the working lumen within overtube **780** may have dividing wall **786** formed within and extending diametrically to form two separate lumens **784, 784'.** Dividing wall **786 is** preferably formed within the distal end portion of overtube **780** and may be as long as windows **782, 782'.** Alternatively, wall **786** may extend throughout the length of overtube **780.** Each separated lumen **784, 784'** may open to a window **782, 782',** respectively. As shown in Fig. 19B, apposed walls of tissue **788, 788'** may be drawn into respective lumens **784, 784'** until they overlap one another between dividing wall **786.** To fasten and/or approximate the apposed walls of tissue **788, 788',** an endoscope or flexible fastening device may be used to fasten the tissue to one another. Alternatively, longitudinally-shaped channels **789** may optionally be defined within wall **786** near where dividing wall **786** joins overtube **780** wall. A helically or spirally-shaped fastener, as described above, may then be used with this variation as well. This variation **780** may of may not be used with a separate endoscope disposed within the working lumen.

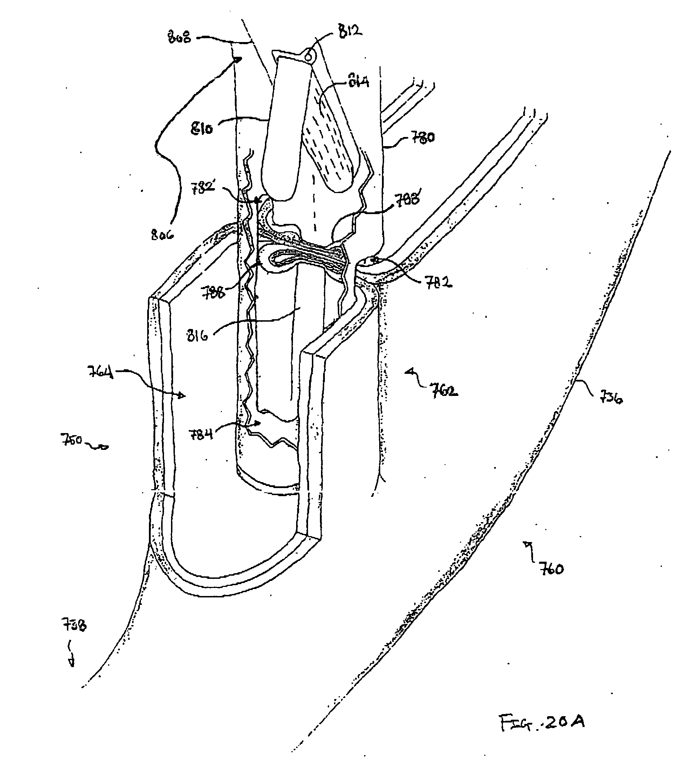

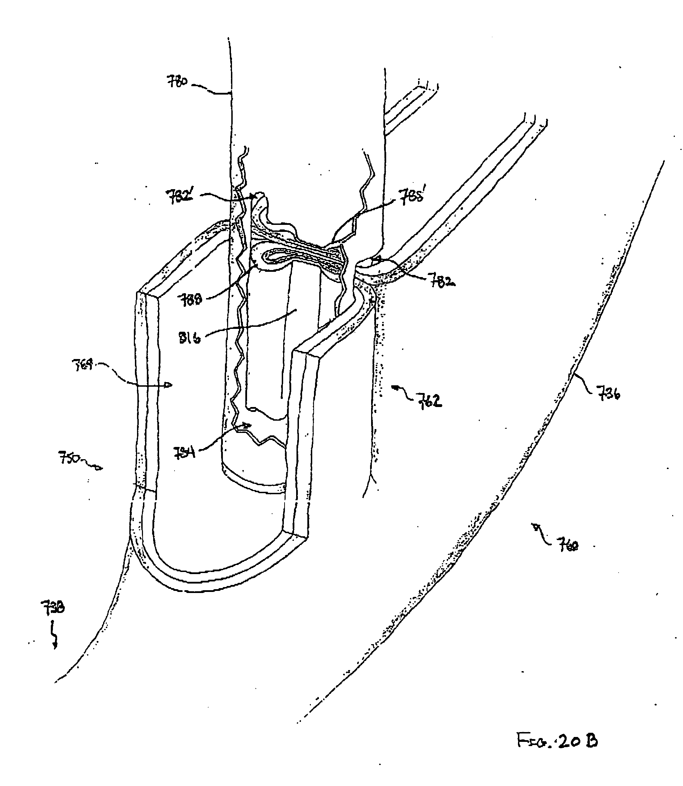

Fig. 20A shows an isometric view of overtube variation **780** inserted within stomach lumen **764** with the walls of both stomach **736** and overtube **780** partially removed for clarity. As shown, the invaginated and overlapping stomach lining **788, 788'** can be seen within first and second lumen **784, 784'** (dividing wall **786** has been removed for clarity only). As the overlapping tissue **788, 788'** is held relative to one another, fastening assembly **806** may be advanced within overtube **780** to where the approximated tissue is positioned. Fastening assembly **806** may be any one of the fastening devices or endoscopic stapling assemblies as described above. The example shown comprises shaft 808 with fastening anvil **810** pivotally attached about pivot **812.** Fastening assembly **806** may be manipulated from its proximal end to clamp the tissue **788, 788'** between anvil **810** and fasteners **814.** As described above, fasteners **814** may be in the form of staples, rivets, or other mechanical fasteners as described herein, which may be housed within shaft **808.** To secure the tissue to one another, fastening assembly **806** may be clamped onto overlapping tissue **788, 788'** to create a fixation zone or region **816.** Fig. 20B shows tissue **788, 788'** having been attached together over fixation region **816** and fastening assembly 806 having been withdrawn from overtube **780.**

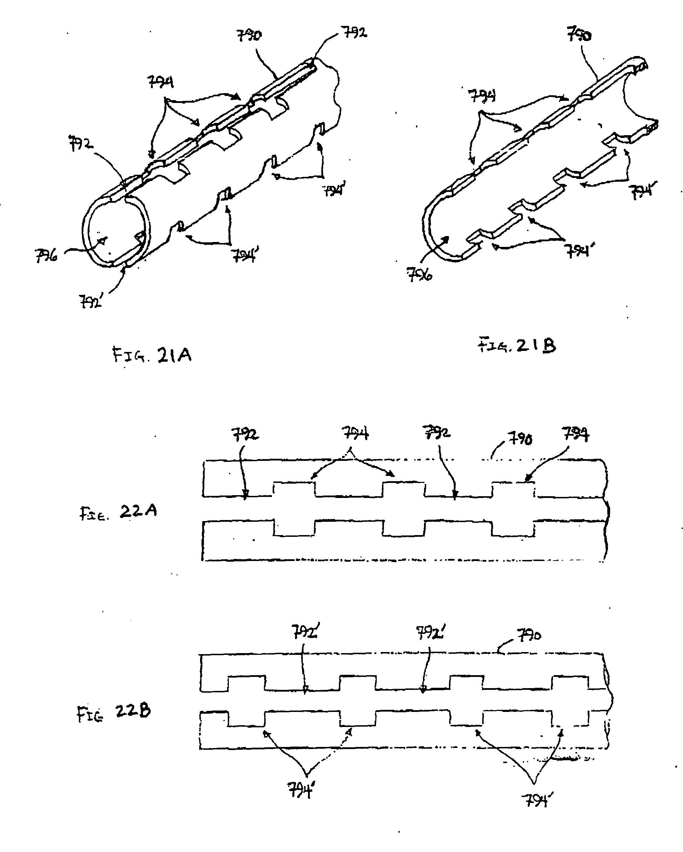

Figs. 21A to 22B show another variation for configuring the distal end of the overtube. Fig. 21A shows an isometric view of overtube **790** which has several alternating windows. On apposing sides of overtube **790,** first channel **792** and second channel **792'** may be defined extending longitudinally along the distal end of overtube **790.** Along first channel **792,** a number of windows **794** may be defined which are preferably evenly spaced from one another with first channel **792** running throughout. On the apposed side, second channel **792'** may be defined with a number of windows **794'** which are also evenly spaced from one another. Windows **794** and **794'** may be similarly shaped but are preferably alternating with apposed windows to allow tissue to be drawn within lumen **796** in an overlapping manner. Fig. 21B shows an isometric view of the overtube **790** from Fig. 21A with half the wall removed for clarity. Fig. 22A shows a side view of overtube **790** and first channel **792** while Fig. 22B shows a side view of the other side of overtube **790** and second channel **792'.** The number of windows in either channel **792, 792** is not intended to be limiting but any number of alternating windows may be incorporated within overtube **790** depending upon the desired application and results.

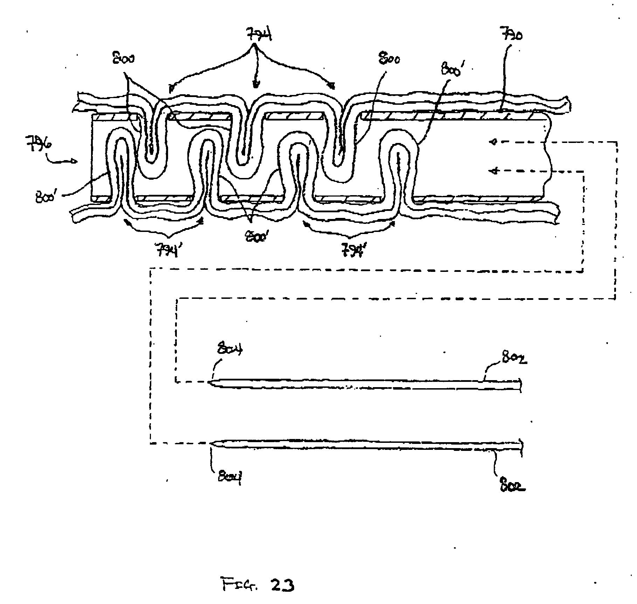

Fig. 23 shows a cross-sectioned side view of overtube **790** from Figs. 21A to 22B showing the invagination of tissue within. As seen, invaginated tissue **800, 800'** may be drawn into lumen **796** through the respective first and second windows **794, 794'.** Once drawn within, the overlapping tissue **800, 800'** may be fastened to one another using spiral fastener **714** as described above using a drive tube. Alternatively, and as shown, fasteners **802,** each preferably having tapered or sharpened distal tips **804,** may be driven distally from the proximal end of overtube **790** into each adjacent region of invaginated tissue **800, 800'** to fasten them together. Once fastened, overtube **790** may then be withdrawn from the area by simply retracting overtube **790** while maneuvering the fastened tissue through first and second channels **792, 792'.** Although two fasteners **802** are shown, any number of fasteners may be utilize depending upon the desired results. Additionally, to aid in preventing the fasteners from backing out of the tissue or from being dislodged, barbs or whisker-like filaments may be formed along its length to protrude and to engage the tissue once the fasteners are in place within the tissue.

## Claims

1. A device for reducing the interior size of a stomach (10) by dividing the stomach into a main pouch (20) and a modified pouch (22), the device comprising a tubular approximating device (280) having a proximal end and an open distal end with a length therebetween and defining therein a common channel (284), wherein:
the tubular approximating device is constructed and arranged to be connected to a means for creating a vacuum in the common channel;
the open distal end of the tubular approximating device is plugged with a removeable plug (292);
the tubular approximating device has a slotted first vacuum port (286) aligned parallel to a longitudinal axis of the device and for releasably adhering a first fold of stomach tissue to the slotted first vacuum port using a vacuum and a slotted second vacuum port (286) aligned parallel to the longitudinal axis of the device and opposite to the slotted first vacuum port and for releasably adhering a second fold of the stomach tissue to the slotted second vacuum port using a vacuum so as to enable the first and second folds of stomach tissue to be releasably positioned near to one another;
the slotted vacuum ports (286) extend proximally from the open distal end of the tubular approximating device;
the device further comprises a fastener (288, 289) disposed within the tubular approximating device (280), the fastener being deployable therefrom and constructed and arranged to fasten together the first and second folds of stomach tissue to form the modified pouch (22) and the main pouch (20) from the interior of the stomach (10);
the fastener (288, 289) is either: a clip (288) with two parallel legs each provided with a piercing end (290); or is a spiral coil (289) provided with a piercing distal end (291) and reducing in diameter in the proximal direction from a diameter the same as, or slightly less than, the inner diameter of the tubular approximating device (280); and
the device yet further comprises means for urging the clip (288) through the tubular approximating device (280) in the distal direction or for rotating the spiral coil (289).

2. The device of claim 1 wherein the first and second vacuum ports (286) are in fluid communication with the common channel (284) defined within the tissue adhering member (280).

3. The device of claim 1 or claim 2 wherein, when the fastener is the spiral coil (289), the coil (289) is configured to alternatingly pierce the first and second folds of stomach tissue while approximating the first and second folds of stomach tissue together.

4. The device of any one of the preceding claims wherein the length of the tubular approximating device (280) is straight.

5. The device of any one of the preceding claims wherein the tubular approximating device (280) is adapted to be inserted into the stomach endoscopically via an esophageal passageway.

6. The device of any one of the preceding claims wherein the fastener (288, 289) comprises a biocompatible material.

7. The device of claim 6 wherein the biocompatible material is selected from the group consisting of polymers and superelastic alloys.

8. The device of any one of the preceding claims wherein, when the fastener is the spiral coil (289), the device further comprises a plurality of additional said spiral coils (289), each of said spiral coils being configured to fasten the first and second folds of stomach tissue while juxtaposed linearly to form a continuous fixation line.

## Patentansprüche

1. Eine Vorrichtung zur Verminderung der Innengröße eines Magens (10) durch Teilen des Magens in eine Haupttasche (20) und eine modifizierte Tasche (22), wobei die Vorrichtung eine schlauchröhrenförmige Approximationsvorrichtung (280) mit einem proximalen Ende und einem offenen distalen Ende mit einer Länge dazwischen und darin definierend einen gemeinsamen Kanal (284) umfasst, wobei:
die schlauchröhrenförmige Approximationsvorrichtung zur Verbindung mit einem Mittel zur Erzeugung eines Vakuums in dem gemeinsamen Kanal konstruiert und angeordnet ist;
auf das offene Ende der schlauchröhrenförmigen Approximationsvorrichtung eine abnehmbare Aufsteckvorrichtung (292) aufgesteckt ist;
die schlauchröhrenförmige Approximationsvorrichtung eine geschlitzte erste Vakuumöffnung (286), die parallel zu einer Längsachse der Vorrichtung und zum wieder ablösbaren Anheften einer ersten Falte von Magengewebe an die geschlitzte erste Vakuumöffnung unter Anwendung eines Vakuums ausgerichtet ist, und eine geschlitzte zweite Vakuumöffnung (286), die parallel zu der Längsachse der Vorrichtung und gegenüber der ersten geschlitzten Vakuumöffnung und zum wieder ablösbaren Anheften einer zweiten Falte des Magengewebes an die geschlitzte zweite Vakuumöffnung unter Verwendung eines Vakuums ausgerichtet ist, aufweist, so dass sich die erste Falte und die zweite Falte des Magengewebes wieder ablösbar nahe beieinander anordnen lassen;
die geschlitzten Vakuumöffnungen (286) sich proximal von dem offenen distalen Ende der schlauchröhrenförmigen Approximationsvorrichtung erstrecken;
die Vorrichtung weiterhin ein Verbindungselement (288, 289) umfasst, das innerhalb der schlauchröhrenförmigen Approximationsvorrichtung (280) angeordnet ist, wobei das Verbindungselement davon entfaltbar ist und konstruiert und angeordnet ist, um die erste Falte und die zweite Falte des Magengewebes miteinander unter Bildung der modifizierten Tasche (20) aus dem Inneren des Magens (10) zu verbinden;
das Verbindungselement (288, 289) eines von folgendem ist: eine Klammer (288) mit zwei parallelen Schenkeln, die jeweils mit einem Lochdom-Ende (290) ausgestattet sind; oder es ist eine Spiralfeder (289), die mit einem distalen Lochdom-Ende (291) ausgestattet ist und im Durchmesser in proximaler Richtung von einem Durchmesser abnehmend ist, der gleich oder etwas kleiner ist als der Innendurchmesser der schlauchröhrenförmigen Approximationsvorrichtung (280); und
die Vorrichtung weiterhin noch Mittel zum Drücken der Klammer (288) durch die schlauchröhrenförmige Approximationsvorrichtung (280) in distaler Richtung oder zum Drehen der Spiralfeder (289) umfasst.

2. Die Vorrichtung nach Anspruch 1, wobei die erste und die zweite Vakuumöffnung (286) in fluider Kommunikation mit dem gemeinsamen Kanal (284) stehen, der in dem Gewebe-Verknüpfungselement (280) definiert ist.

3. Die Vorrichtung nach Anspruch 1 oder Anspruch 2, wobei, wenn das Verbindungselement die Spiralfeder (289) ist, die Spirale (289) zum alternierenden Durchstoßen der ersten Falte und der zweiten Falte von Magengewebe während der Annäherung der ersten Falte und der zweiten Falte von Magengewebe aneinander konfiguriert ist.

4. Die Vorrichtung nach einem der vorhergehenden Ansprüche, wobei die Länge der schlauchröhrenförmigen Approximationsvorrichtung (280) gerade ist.

5. Die Vorrichtung nach einem der vorhergehenden Ansprüche, wobei die schlauchröhrenförmige Approximationsvorrichtung (280) dazu geeignet ist, über einen ösophagealen Weg endoskopisch in den Magen eingeführt zu werden.

6. Die Vorrichtung nach einem der vorhergehenden Ansprüche, wobei das Verbindungselement (288, 289) ein biokompatibles Material einschließt,

7. Die Vorrichtung nach Anspruch 6, wobei das biokompatible Material aus der Gruppe ausgewählt ist, die aus Polymeren und superelastischen Legierungen besteht.

8. Die Vorrichtung nach einem der vorhergehenden Ansprüche, wobei das Verbindungselement die Spiralfeder (289) ist, die Vorrichtung weiterhin eine Vielzahl von zusätzlichen der Spiralfedern (289) umfasst, wobei die Spiralfedern jeweils konzipiert sind, um die erste und die zweite Falte von Magengewebe zu verbinden, während sie linear nebeneinander liegen, um eine kontinuierliche Fixationslinie zu bilden.

## Revendications

1. Dispositif pour réduire la taille intérieure d'un estomac (10) en divisant l'estomac en une poche principale (20) et une poche modifiée (22), le dispositif comprenant un dispositif de rapprochement tubulaire (280) ayant une extrémité proximale et une extrémité distale ouverte avec une longueur entre celles-ci et définissant à l'intérieur un canal commun (284), dans lequel :
le dispositif de rapprochement tubulaire est construit et conçu, pour être raccordé à des moyens pour créer un vide dans le canal commun ;
l'extrémité distale ouverte du dispositif de rapprochement tubulaire est obturée à l'aide d'un bouchon amovible (292) ;
le dispositif de rapprochement tubulaire a un premier orifice sous vide fendu (286) aligné parallèlement à un axe longitudinal du dispositif et fait adhérer de manière séparable un premier pli de tissu stomacal au premier orifice sous vide fendu en utilisant du vide, et a un second orifice sous vide fendu (286) aligné parallèlement à l'axe longitudinal du dispositif et opposé au premier orifice sous vide fendu et fait adhérer de manière séparable un second pli de tissu stomacal au second orifice sous vide fendu en utilisant du vide, de manière à permettre de positionner les premier et second plis de tissu stomacal de manière séparable l'un près de l'autre ;
les orifices sous vide fendus (286) s'étendent de manière proximale à partir de l'extrémité distale ouverte du dispositif de rapprochement tubulaire ;
le dispositif comprend en outre une attache (288, 289) disposée à l'intérieur du dispositif de rapprochement tubulaire (280), l'attache pouvant être déployée à partir de celui-ci et étant construite et conçue pour attacher ensemble les premier et second plis de tissu stomacal pour former la poche modifiée (22) et la poche principale (20) à partir de l'intérieur de l'estomac (10) ;
l'attache (288, 289) est : une pince (288) ayant deux pattes parallèles chacune munie d'une extrémité de percement (290) ; ou est une spirale hélicoïdale (289) munie d'une extrémité distale de percement (291) et diminuant en diamètre dans la direction proximale à partir d'un diamètre identique, ou légèrement inférieur, au diamètre intérieur du dispositif de rapprochement tubulaire (280) ; et
le dispositif comprend encore en plus des moyens pour pousser la pince (288) à travers le dispositif de rapprochement tubulaire (280) dans la direction distale ou pour faire tourner la spirale hélicoïdale (289).

2. Dispositif selon la revendication 1 dans lequel les premier et second orifices sous vide (286) sont en communication fluidique avec le canal commun (284) défini à l'intérieur de l'élément adhérant au tissu (280).

3. Dispositif selon la revendication 1 ou la revendication 2 dans lequel, lorsque l'attache est une spirale hélicoïdale (289), la spirale (289) est configurée pour percer alternativement les premier et second plis de tissu stomacal tout en rapprochant les premier et second plis de tissu stomacal en même temps.

4. Dispositif selon l'une quelconque des revendications précédentes dans lequel la longueur du dispositif de rapprochement tubulaire (280) est rectiligne.

5. Dispositif selon l'une quelconque des revendications précédentes dans lequel le dispositif de rapprochement tubulaire (280) est adapté pour être inséré dans l'estomac par voie endoscopique via un passage oesophagien.

6. Dispositif selon l'une quelconque des revendications précédentes dans lequel l'attache (288, 289) comprend un matériau biocompatible.

7. Dispositif selon la revendication 6 dans lequel le matériau biocompatible est choisi parmi le groupe constitué de polymères et d'alliages superélastiques.

8. Dispositif selon l'une quelconque des revendications précédentes dans lequel, lorsque l'attache est la spirale hélicoïdale (289), le dispositif comprend en outre une pluralité de spirales hélicoïdales supplémentaires (289), chacune desdites spirales hélicoïdales étant configurée pour fixer les premier et second plis de tissu stomacal tout étant juxtaposée linéairement pour former une ligne de fixation continue.
